# NEUE EUROPÄISCHE PATENTSCHRIFT

(11) **EP 1 230 209 B3**
(45) Veröffentlichungstag dieser Patentschrift: **05.10.2011**
(45) Hinweis auf die Patenterteilung: 12.01.2005
(21) Anmeldenummer: 00989857.8
(22) Anmeldetag: 15.11.2000
(51) Int. Cl.: C07C 215/54

(54) **STABILE SALZE NEUARTIGER DERIVATE VON 3,3-DIPHENYLPROPYLAMINEN**
STABLE SALTS OF NOVEL DERIVATIVES OF 3,3-DIPHENYLPROPYLAMINES
SELS STABLES DE NOUVEAUX DERIVES DE 3,3-DIPHENYLPROPYLAMINES

(30) Priorität: 16.11.1999 DE 19955190
(43) Veröffentlichungstag der Anmeldung: 14.08.2002
(60) Teilanmeldung: 04018487.1
(73) Patentinhaber: UCB Pharma GmbH, 40789 Monheim (DE)
(72) Erfinder: MEESE, Claus, 40789 Monheim (DE)
(74) Vertreter: Bausch, Thorsten
(86) Internationale Anmeldenummer: PCT/EP2000/011309
(87) Internationale Veröffentlichungsnummer: WO 2001/035957

(56) Entgegenhaltungen:
- WO-A-94/11337
- WO-A-98/43942
- PALMER, L. ET AL.: "Determination of tolterodine and the 5-hydroxymetylmetabolite ..." J. PHARM. BIOMED. ANAL., Bd. 16, 1997, Seiten 155-165, XP000995770

## Beschreibung

Die vorliegende Erfindung betrifft hochreine, kristalline, stabile Verbindungen neuartiger Derivate von 3,3-Diphenylpropylaminen in Form ihrer Salze und Verfahren zu deren Herstellung.

Aus WO 99/58478 (PCT/EP99/03212) sind neuartige Derivate von 3,3-Diphenylpropylaminen bekannt, und R-(+)-Isobuttersäure-2-(3-diisopropylamino-1-phenylpropyl)-4-hydroxymethylphenylester Hydrochlorid in Form eines farblosen hygroskopischem Feststoffs ist auf Seite 62, Zeilen 13-24 offenbart.

Sie sind wertvolle Prodrugs für die Behandlung von Hamdrang-Inkontinenz und anderen spasmogenen Leiden, die den Nachteil bisher zur Verfügung stehender Wirkstoffe, nämlich zu geringe Absorption der Wirkstoffe durch biologische Membranen oder deren ungünstigen Metabolismus vermeiden.

Weiterhin zeichnen sich diese neuartigen Prodrugs durch verbesserte pharmakokinetische Eigenschaften im Vergleich zu Oxybutynin und Tolterodin aus.

Bevorzugte Verbindungen aus der Gruppe dieser neuartigen Derivate von 3,3-Diphenylpropylaminen sind Ester aliphatischer oder aromatischer Carbonsäuren mit der nachfolgend genannten allgemeinen Formel A 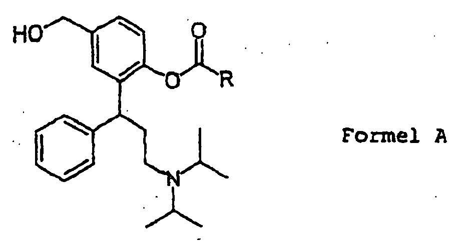 in der R die Bedeutung von C₁-C₆-alkyl, C₃-C₁₀-cycloalkyl oder unsubstituiertem oder substituiertem Phenyl hat. Sie können in Form ihrer optischen isomere, als Racematengemisch und in Form ihrer individuellen Enantiomere vorliegen.

Verbindungen der Struktur der Formel A besitzen allerdings eine geringe Wasseriöslichkelt. Diese verringert ihre orale Bioverfügbarkeit.

Schließlich neigen Monoester der Struktur, wie sie in Formel A wiedergegeben sind, zu Intermolekularer Umesterung.

Bei längerer Lagerung ist deshalb unter Gehaltsabnahme von Verbindungen der Struktur der allgemeinen Formel A eine Zunahme von Diester und freiem Diol feststellbar.

Zwar lassen sich grundsätzlich Salze der Verbindungen der allgemeinen Formel A erhalten, indem Lösungen der Verbindungen der Formel A (Basenteil) mit Lösungen von Säuren in jeweils geeigneten Lösungsmitteln vereinigt werden, jedoch erweisen sich die als Festkörper erhaltenen Salze als durchweg amorph und/oder hygroskopisch und sind auch aus den üblichen Lösungsmitteln nicht ohne weiteres kristallisierbar. Derartige Salze weisen eine zu geringe chemische Stabilität auf, um als wertvolle pharmazeutische Wirkstoffe galenisch verarbeitet werden zu können.

Überraschenderweise wurde nun gefunden, daß sich die vorgenannten Nachteile vermeiden lassen, wenn Verbindungen der Struktur der allgemeinen Formel A, nachdem sie unter spezieller Reaktionsführung dargestellt wurden, mit einer physiologisch verträglichen anorganischen oder organischen Säure der allgemeinen Formel H-X, in der X den jeweiligen Säurerest bedeutet, zu ihrem jeweiligen Salz der allgemeinen Formel I umgesetzt werden.

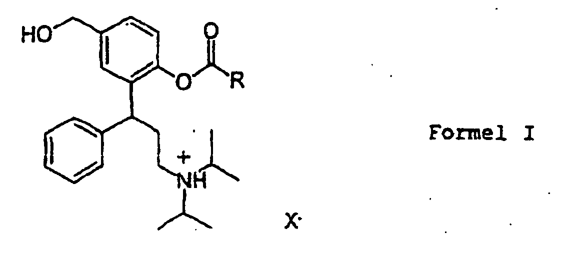

Es ist daher Aufgabe der vorliegenden Erfindung, hochreine, kristalline, stabile Verbindugen neuartiger Derivate von 3,3-Diphenytpmpylaminen in Form ihrer Salze zur Verfügung zu stellen, die die erwähnten Nachteile vermeiden und sich besonders gut zum Einsatz in pharmazeutisch-technischen Formulierungen eignen und zu solchen verarbeiten lassen.

Eine weitere Aufgabe der vorliegenden Erfindung ist es, ein Verfahren zur Herstellung derartiger hochreiner, kristalliner, stabiler Verbindungen in Form ihrer Salze zur Verfügung zu stellen.

Schließlich ist es Aufgabe der Erfindung, ein Verfahren zur Herstellung der vorgenannten Verbindungen zur Verfügung zu stellen, mit dem die Verfahrensprodukte und die jeweiligen Zwischenprodukte chemo- und regioselektiv in hoher Ausbeute erhalten werden.

Diese Aufgabe wurde dadurch gelöst, daß hochreine, kristalline, stabile Verbindungen der 3,3-Diphenylpropylamine in Form ihrer Salze der allgemeinen Formel I zur Verfügung gestellt werden, 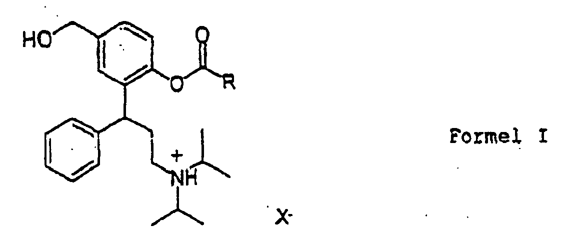 worin R für C₁-C₆-alkyl, C₃-C₁₀-cycloalkyl, substituiertes oder unsubstituiertes Phenyl steht und X⁻ der Säurerest einer physiologisch verträglichen anorganischen oder organischen Säure ist, mit der Maßgabe, dass die Verbindung nicht R-(+)-Isobuttersäure-2-(3-diisopropylamino-1-phenylpropyl)-4-hydroxymethylphenylester Hydrochlorid ist.

Nach einer Ausführung der Erfindung können die Salze der allgemeinen Formel I den jeweiligen Säurerest X⁻ der nachfolgend genannten Säuren enthalten:

Salzsäure, Bromwasserstoftsäure, Phosphorsäure, Schwefelsäure, Salpetersäure, Essigsäure, Propionsäure, Palmitinsäure, Stearinsäure, Maleinsäure, Fumarsäure, Oxalsäure, Bernsteinsäure, DL-Äpfelsäure, L-(-)-Äpfelsäure, D-(+)-Äpfelsäure, DL-Weinsäure, L-(+)-Weinsäure, D-(-)-Weinsäure, Citronensäure, L-Asparaginsäure, L-(+)-Ascorbinsäure, D-(+)-Glucuronsäure, 2-Oxopropionsäura (Brenztraubensäure), Furan-2-carbonsäure (Brenzschleimsäure), Benzoesäure, 4-Hydroxybenzoesäure, Salicylsäure, Vanillinsäure, 4-Hydroxyzimtsäure, Gallussäure, Hippursäure (N-Benzoyl-glycin), Acetursäure (N-Acetytglycin), Phloretinsäure (3-(4-Hydroxypheny))-propionsäure), Phthalsäure, Methansulphonsäure oder Orotsäure.

Nach einer weiteren Ausführungsform der Erfindung werden R-konfigurierte Verbindungen der allgemeinen Formel 2 zur Verfügung gestellt, 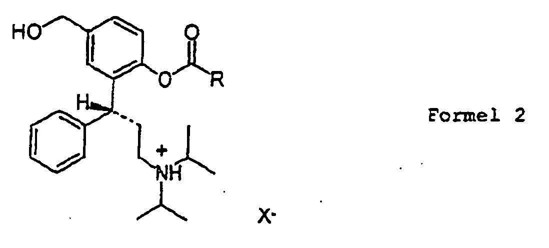 worin R für C₁-C₆-alkyl, C₃-C₁₀-cycloalkyl, substituiertes oder unsubstituiertes Phenyl steht und X⁻ der Säurerest einer physiologisch verträglichen anorganischen oder organischen Säure Ist.

Nach einer vorteilhaften Ausführungsform der Erfindung können die Verbindungen in Form ihrer Salze der allgemeinen Formel 2 den jeweiligen Säurerest X⁻ der nachfolgend genannten Säuren enthalten:

Salzsäure, Bromwasserstoffsäure, Phosphorsäure, Schwefelsäure, Salpetersäure, Essigsäure. Propionsäure, Palmitinsäure, Stearinsäure, Maleinsäure, Fumarsäure, Oxalsäure, Bernsteinsäure, DL-Äpfelsäure, L-(-)-Äpfelsäure, D-(+)-Äpfelsäure, DL-Weinsäure, L-(+)-Weinsäure, D-(-)-Weinsäure, Citronensäure, L-Asparaginsäure, L-(+)-Ascorbinsäure, D-(+)-Glucuronsäure, 2-Oxopropionsäure (Brenztraubensäure), Furan-2-carbonsäure (Brenzschleimsäure), Benzoesäure, 4-Hydroxybenzoesäure, Salicylsäure, Vanillinsäure, 4-Hydroxyzimtsäure, Gallussäure, Hippursäure (N-Benzoyl-glycin), Acetursäure (N-Acetylglycin), Phloretinsäure (3-(4-Hydroxyphenyl)-propionsäure), Phthalsäure, Methansulphonsäure oder der Orotsäure ist.

Bevorzugte Verbindungen der vorliegenden Erfindung sind die salze
- R-(+)-2-(3-Diisopropylamino-1-phenylpropyl)-4-hydroxymethylphenylisobuttersäureester Hydrogenfumarat und
- R-(+)-2-(3-Diisopropylamino-1-phenylpropyl)-4-hydroxymethylphenylisobuttersäureester Hydrochlorid Hydrat.

Weiterhin sind solche Verbindungen bevorzugt, worin R für Cyclopropyl, Cyclobutyl, Cyclopentyl, Cyclohexyl, 4- (1-Cyclopropyl-methanoyloxy)-phenyl, 4-(1-Cyclobutyl-methanoyloxy)-phenyl, 4-(1-Cyclohexyl-methanoyloxy)-phenyl oder 4-(2,2-Dimethyl-propanoyloxy)-phenyl steht und X⁻ Chlorid bedeutet.

Bevorzugt sind insbesondere [(R)-3-(2-(1-[4-(1-Cyclopropylmethanoyloxy)-phenyl)-methanoyloxyl-5-hydroxymethyl-phenyl)-3-phenylpropyl]-diisopropyl-ammoniumchlorid, [(R)-3-(2-{1-[4-(1-Cyclobutyl-methanoyloxy) -phenyl]-methanoyioxy}-5-hydroxymethyl-phenyl)-3-phenyl-propyl]-diisopropyl-ammoniumchlorid,[(R)-3-(2-{1-[4-(1-Cyclohexyl-methanoyloxy)-phenyl]-methanoyloxy)-5-hydroxymethyl-phenyl)-3-phenyl-propyl]-diisopropylammoniumchlorid, [(R)-3-(2-(1-[4-(2,2-Dimethyl-propanoyloxy)-phenyl]-methanoyloxy}-5-hydroxymethyl-phenyl)-3-phenyl-propyl]-diisopropyl-ammoniumchlorid, {(R)-3-[2-(1-Cyclopropylmethanoyloxy)-5-hydroxymethyl-phenyl]-3-pheny3-propyl}-diisopropyl-ammoniumchlorid, ((R)-3-[2-(1-Cyclobutyl-methanoyloxy) -5-hydroxymethyl-phenyl]-3-phenyl-propyl}-diisopropylammoniumchlorid, {(R)-3-[2-(1-Cyclopentyl-methanoyloxy)-5-hydroxymethyl-phenyl]-3-phenyl-propyl}-diisopropyl-ammoniumchlorid, ((R)-3-[2-(1-Gyclohexyl-methanoyloxy)-5-hydroxymethyl-phenyl)-3-phenyl-propyl)-diisopropyl-ammoniumchlorid.

In den Verbindungen der vorliegenden Erfindung bedeutet der Ausdruck "alkyl" vorzugsweise eine geradkettige oder verzweigtkettige Kohlenwasserstoffgruppe mit 1 bis 6 C-Atomen. Besonders bevorzugt sind Methyl, Ethyl, Propyl, Isopropyl, Butyl, Isobutyl, Pentyl und Hexyl. Der Ausdruck "Cycloalkyl" bezeichnet zyklische Kohlenwasserstoffgruppen, die 3 bis 10 Kohlenstoffatome aufweisen, die auch geeignete Substituenten anstelle der Wasserstoffatome enthalten können.

Der Ausdruck "Phenyl" bezeichnet eine -C₆H₅-Gruppe, die substituiert oder unsubstituiert sein kann. Geeignete Substituenten können beispielsweise Alkyl, Alkoxy, Halogen, Nitro und Amin sein. Der Ausdruck "Alkoxy" hat, bezogen auf den Alkylteil, die gleiche Bedeutung, wie sie bereits oben für "alkyl" angegeben wurde. Geeignete Halogene sind Fluor-, Chlor-, Brom- und Iodatome.

Die vorliegende Erfindung umfaßt auch Verfahren zur Herstellung der erfindungsgemäßen Verbindungen der allgemeinen Formel I.

Das Verfahren zeichnet sich durch Chemo- und Regioselektivität sowie hohe Ausbeute aus.

Verbindungen der allgemeinen Formel I 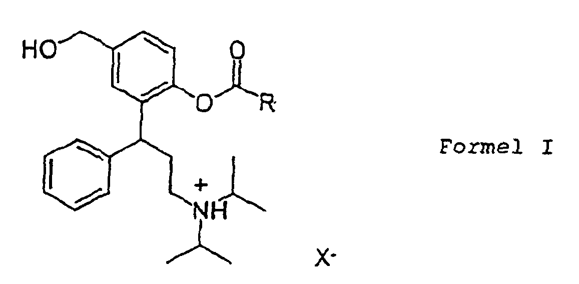 worin R für C₁-C₆-alkyl, C₃-C₁₀-cycloalkyl, substituiertes oder unsubstituiertes Phenyl steht und X⁻ der Säurerest einer physiologisch verträglichen anorganischen oder organischen Säure steht, werden hergestellt, indem
a) eine Verbindung der Formel III 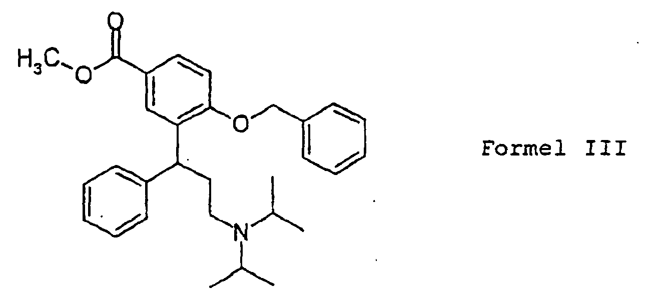 mit einem Hydrierungsmittel zur Bildung einer Verbindung der Formel V 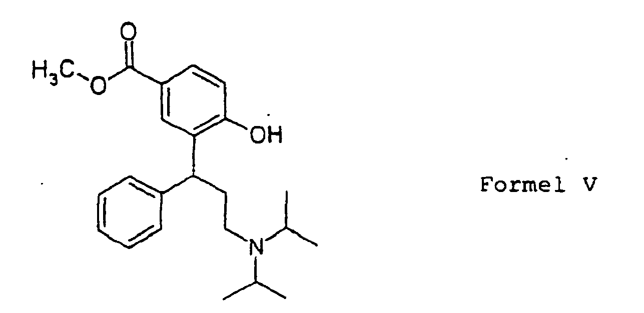 gespalten wird, worauf
b) die so erhaltene Verbindung der Formel V mit einem Reduktionsmittel umgesetzt wird, um eine Verbindung der Formel VI 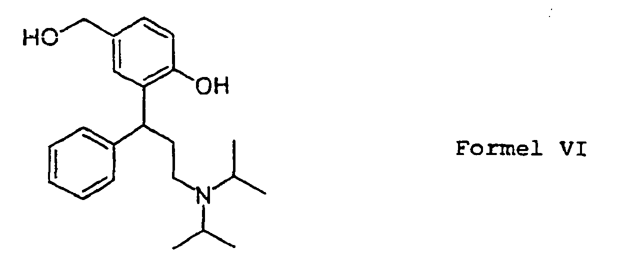 zu ergeben, die
c) mit einem Acylierungsmittel umgesetzt wird, um eine Verbindung der Formel A 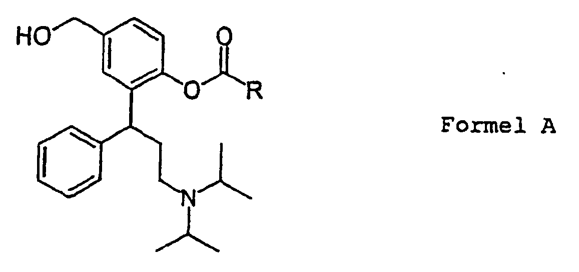 zu erhalten, worin R die oben genannte Bedeutung hat, die
d) mit einer physiologisch verträglichen anorganischen oder organischen Säure unter Bildung einer Verbindung der Formel I 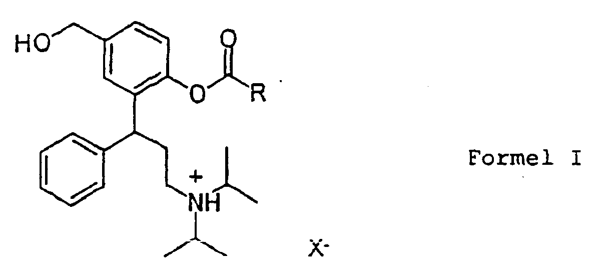 umgesetzt wird, worin R für C₁-C₆ alkyl, C₃-C₁₀ cycloalkyl, unsubstituiertes oder substituiertes Phenyl steht und X⁻ für den Säurerest einer physiologisch verträglichen anorganischen oder organischen Säure steht.

Verfahrensgemäß werden zur Herstellung der Verbindungen der allgemeinen Formel die Säuren Salzsäure, Bromwasserstoffsäure, Phosphorsäure, Schwefelsäure, Salpetersäure, Essigsäure, Propionsäure, Palmitinsäure, Stearinsäure, Maleinsäure, Fumarsäure, Oxalsäure, Bernsteinsäure, DL-Apfelsäure, L-(-)-Apfelsäure, D-(+)-Äpfelsäure, DL-Weinsäure, L-(+)-Weinsäure, D-(-)-Weinsäure, Citronensäure, L-Asparaginsäure, L-(+)-Ascorbinsäure, D-(+) -Glucuronsäure, 2-Oxopropionsäure (Brenztraubensäure), Furan-2-carbonsäure (Brenzschleimsäure), Benzoesäure, 4-Hydroxybenzoesäure, Salicylsäure, Vanillinsäure, 4-Hydroxyzimtsäure, Gallussäure, Hippursäure (N-Benzoyl-glycin), Acetursäure (N-Acetylglycin), Phloretinsäure (3-(4-Hydroxyphenyl)-propionsäure), Phthalsäure, Methansulphonsäure oder Orotsäure verwendet.

Nach einer vorteilhaften Weiterbildung der Erfindung wird ein Verfahren zur Herstellung von R-konfigurierten Verbindungen der allgemeinen Formel 2 beschrieben, 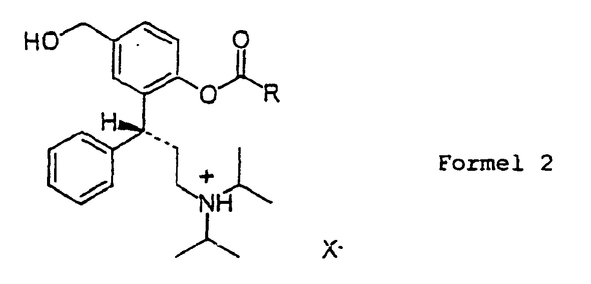 worin R für C₁-C₆ alkyl, C₃-C₁₀ cycloalkyl, substituiertes oder unsubstituiertes Phenyl steht und X- für den Säurerest einer physiologisch verträglichen anorganischen oder organischen Säure steht, indem
a) eine Verbindung der Formel 3 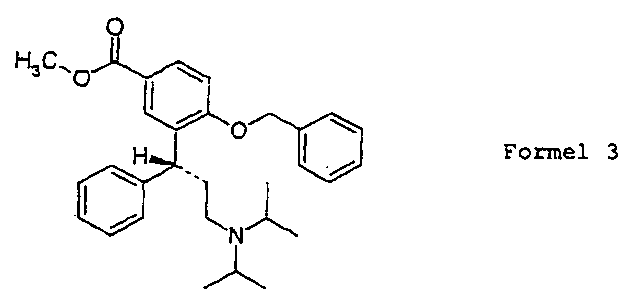 mit einem Hydrierungsmittel zur Bildung einer Verbindung der Formel 5 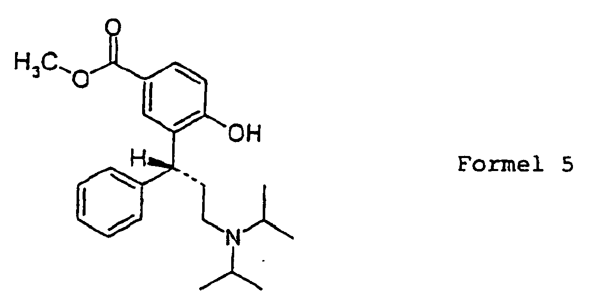 gespalten wird, worauf
b) die so erhaltene Verbindung der Formel 5 mit einem Reduktionsmittel umgesetzt wird, um eine Verbindung der Formel 6 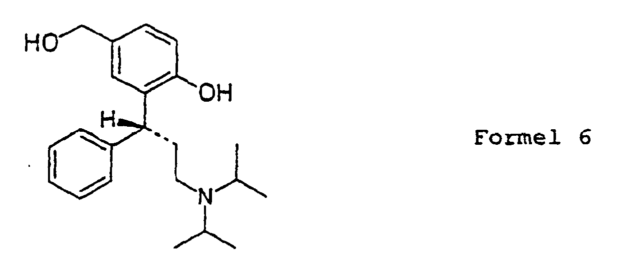 zu ergeben, die
c) mit einem Acylierungsmittel umgesetzt wird, um eine Verbindung der Formel 1 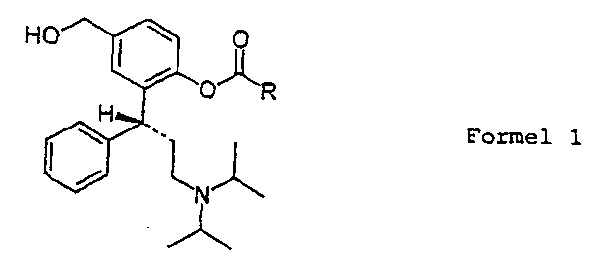 zu erhalten, worin R die oben genannte Bedeutung hat, die
d) mit einer physiologisch verträglichen anorganischen oder organischen Säure unter Bildung einer Verbindung der Formel 2 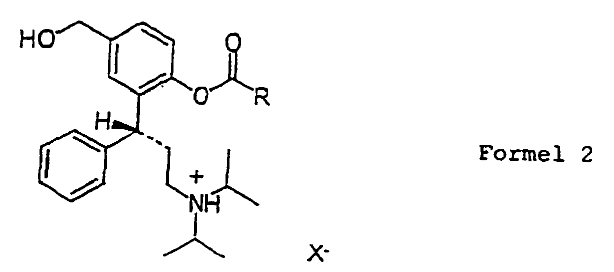 umgesetzt wird, worin R für C₁-C₆-alkyl, C₃-C₁₀-cycloalkyl, unsubstituiertes oder substituiertes Phenyl steht und X⁻ für den Säurerest einer physiologisch verträglichen anorganischen oder organischen Säure steht.

Vorteilhafterweise werden zum Erhalt von Verbindungen der allgemeinen Formel 2 verfahrensgemäß die Säuren Salzsäure, Bromwasserstoffsäure, Phosphorsäure, Schwefelsäure, Salpetersäure, Essigsäure, Propionsäure, Palmitinsäure, Stearinsäure, Maleinsäure, Fumarsäure, Oxalsäure, Bernsteinsäure, DL-Äpfelsäure, L-(-)-Äpfelsäure, D-(+)-Äpfelsäure, DL-Weinsäure, L-(+)-Weinsäure, D-(-)-Weinsäure, Citronensäure, L-Asparaginsäure, L-(+)-Ascorbinsäure, D-(+)-Glucuronsäure, 2-Oxopropionsäure (Brenztraubensäure), Furan-2-carbonsäure (Brenzschleimsäure), Benzoesäure, 4-Hydroxybenzoesäure, Salicylsäure, Vanillinsäure, 4-Hydroxyzimtsäure, Gallussäure, Hippursäure (N-Benzoyl-glycin), Acetursäure (N-Acetylglycin), Phloretinsäure (3-(4-Hydroxyphenyl)-propionsäure), Phthalsäure, Methansulphonsäure oder Orotsäure verwendet.

Besonders vorteilhaft wird, ausgehend von dem kristallinen *R*-(-)-4-Benzyloxy-3-(3-diisopropylamino-1-phenyl-propyl) benzoesäuremethylester, das hochreine, kristalline Zwischenprodukt R-(-)-3-(3-Diisopropylamino-phenylpropyl)-4-hydroxy-benzoesäuremethylester dargestellt, das zu R-(+)-2-(3-Diisopropylamino-1-phenylpropyl)-4-hydroxymethylphenol reduziertwird, schließlich geeignet acyliertwird und anschließend mit einer physiologisch verträglichen anorganischen oder organischen Säure unter spontaner Kristallisation zum jeweiligen hochreinen, kristallinen, stabilen Salz umgesetzt wird.

Je nach verwendetem Säurechlorid werden Verbindungen der allgemeinen Formel 1 erhalten, 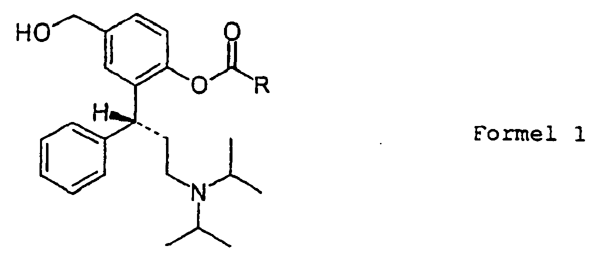 in der R die Bedeutung von C₁-C₆-alkyl, insbesondere Isopropyl, C₃-C₁₀-cycloalkyl oder unsubstituiertem oder substituiertem Phenyl hat.

Zum Erhalt der erfindungsgemäßen Verbindungen in Form ihrer Salze ist die spezielle Reaktionsführung über besondere Zwischenstufen und individualisierbare Zwischenprodukte entscheidend.

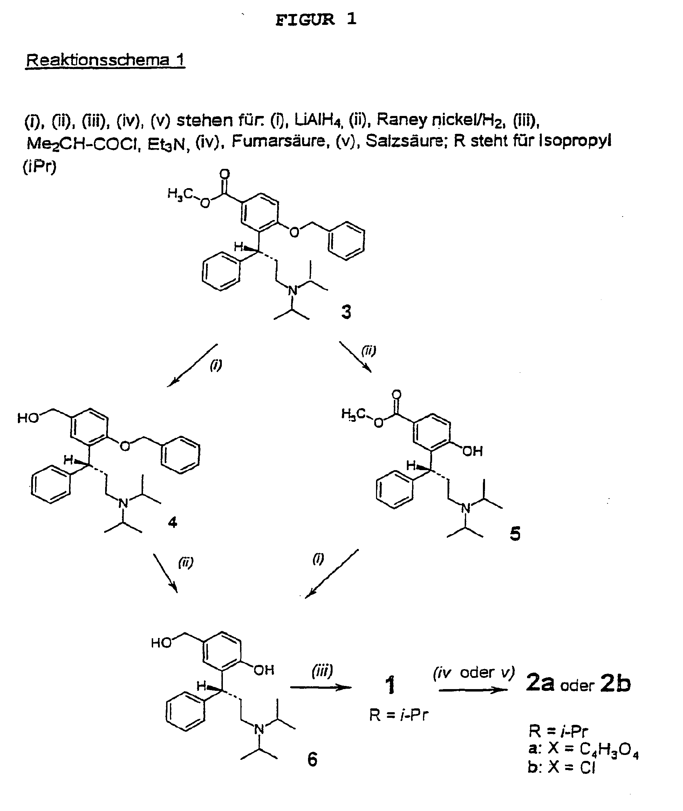

Dies wird anhand des Reaktionsschemas 1 (siehe Figur 1) erläutert, in dem Umsetzungen mit R-konfigurierten Verbindungen, ohne darauf beschränkt zu sein, beschrieben werden.

Darin bedeuten:
3 = R-(-)-4-Benzyloxy-3-(3-diisopropylamino-1-phenylpropyl)-benzoesäure- methylester
4 = R-(+)-[4-Benzyloxy-3-(3-diisopropylamino-1-phenylpropyl)-pheayl]-methanol
5 = R-(-)-3-(3-Diisopropylamino-phenyl-propyl)-4-hydroxybenzoesäuremethylester
6 = R-(+)-2-(3-Diisopropylamino-1-phenylpropyl)-4-hydroxymethylphenol
1 = R-(+)-2-(3-Diisopropylamino-1-phenylpropyl)-4-hydroxymethylphenyl-isobuttersäureester
2a = R-(+)-2-(3-Diisopropylamino-1-phenylpropyl)-4-hydroxymethylphenyl-isobuttersäureester Hydrochlorid Hydrat
2b = R-(+)-2-(3-Diisopropylamino-1-phenylpropyl)-4-hydroxymethylphenyl-isobuttersäureester Hydrogenfumarat

Entsprechend der in den Ausführungsbeispielen erläuterten Reaktionsführung wird die Vorstufe 3 (R-(-)-4-Benzyloxy-3-(3-diisopropylamino-1-phenyl-propyl)-benzoesäure-methylester) kristallin und rein dargestellt.

Vorstufe 3 wird nach üblichen Methoden - z.B. BBr₃, AlCl₃ - vorzugsweise jedoch mittels Wasserstoffgas über Raney-Nickel in Methanol als Lösungsmittel bei Raumtemperatur (RT) zu 5 (R-(-)-3-(3-Diisopropylamino-phenyl-propyl)-4-hydroxy-benzoesäuremethylester) gespalten. Dieses fällt in hochreiner, kristalliner Form (Schmp. 143.7 °C) an.

Schließlich wird 5 mit einem geeigneten Reduktionsmittel - z.B. NaBH₄/EtOH - vorzugsweise LiAlH₄ in einem inerten Lösungsmittel bei niedrigen Temperaturen (-78°C bis + 10°C) reduziert und die Verbindung 6 (R-(+)-2-(3-Diisopropylamino-1-phenylpropyl)-4-hydroxymethylphenol) erhalten. Die Verbindung 6 wird hochrein erhalten und kann aus einem geeigneten Lösungsmittel, wie beispielsweise Ethylacetat, kristallisiert werden. Das farblose feinkristalline Material besitzt einen Schmelzpunkt von 102.3°C. Dies ist insofern überraschend, als die Verbindung 6 im Stand der Technik als amorpher Festkörper beschrieben wird.

Verbindung 6 wird nun in sehr guter Ausbeute und Regio- und Chemoselektivität, zu einem phenolischen Ester acyliert. Diese Reaktion wird bei RT oder niedrigen Temperaturen mit einem Äquivalent Säurechlorid in Gegenwart einer Base in geeigneten Lösungsmitteln ausgeführt. Geeignete Lösungsmittel sind Ethylacetat, Dichlormethan, Tetrahydrofuran, Acetonitril oder Toluol.

Bevorzugt wird die Reaktion mit Isobutyrylchlorid als Säurechlorid und Triethylamin als Base bei den oben angegebenen Temperaturen durchgeführt. Das dann erhaltene 1 (R-(+)-2-(3-Diisopropylamino-1-phenylpropyl)-4-hydroxymethylphenylisobuttersäureester) fällt in so hoher Reinheit an, daß mit Lösungen der Fumarsäure in geeigneten Lösungsmitteln spontane Kristallisation unter Bildung des Hydrogenfumarat-Salzes 2a einsetzt.

Dieses Salz zeigt einen scharfen Schmelzpunkt von 103°C, ist bei RT stabil, nicht hygroskopisch und schließt kein Kristallösemittel ein. Es läßt sich beliebig oft umkristallisieren.

Wird anstatt Fumarsäure wasserfreie Salzsäure - z.B. als etherische Lösung - verwendet, tritt ebenfalls Salzbildung unter Erhalt des kristallinen Produktes 2b (R-(+)-2-(3-Diisopropylamino-1-phenylpropyl) -4-hydroxymethylphenylisobuttersäureester Hydrochlorid Hydrat ein.

Nach erneuter Umkristallisation weist das Produkt 2b einen Schmelzpunktsbereich von 97 - 106°C auf.

Schließlich kann das Produkt 2b ganz besonders vorteilhaft durch die folgende Variante der inversen Reaktionsführung, ausgehend von der Verbindung 6 des Reaktionsschemas 1 direkt erhalten werden. Das Produkt 2b ist damit ohne Zusatz einer externen säure-fangenden Base erhältlich, wie nachfolgend erläutert wird.

Lösungen von 6 (R-(+)-2-(3-Diisopropylamino-1-phenylpropyl)-4-hydroxymethylphenol) werden in Lösungen von Isobuttersäurechlorid getropft, so daß unter geeigneten Polaritätsbedingungen rasch das wasserfreie Produkt 2b auskristallisiert. 2b ist sehr hygroskopisch.

Wird die vorgenannte Reaktion in feuchten Lösungsmitteln durchgeführt, die mindestens ein Moläquivalent Wasser enthalten, wird direkt ein stabiles und kristallines, hydrathaltiges Produkt 2b erhalten, das die oben genannten Schmelzeigenschaften aufweist.

Die erfindungsgemäßen Verbindungen der allgemeinen Formeln I und 2 eignen sich als Schüttgut.

Besonders vorteilhaft sind die hochrein erhältlichen Verbindungen der allgemeinen Formeln III, V, VI, 3, 5, 6 und 7. 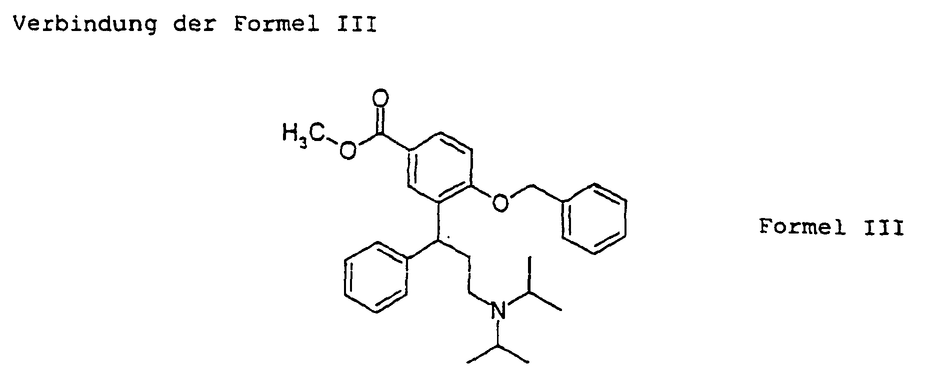 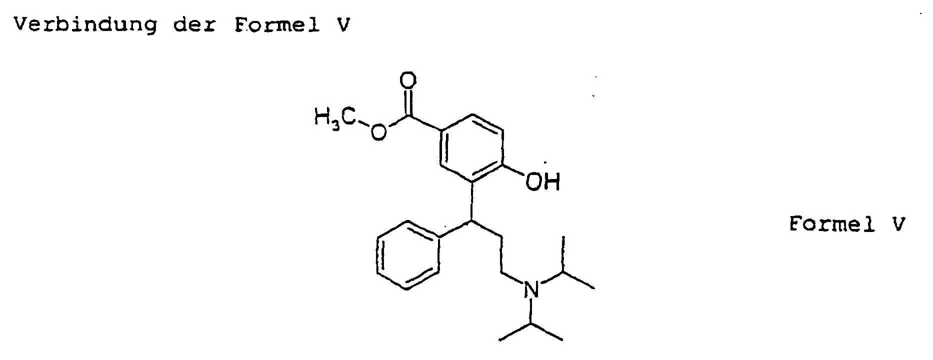 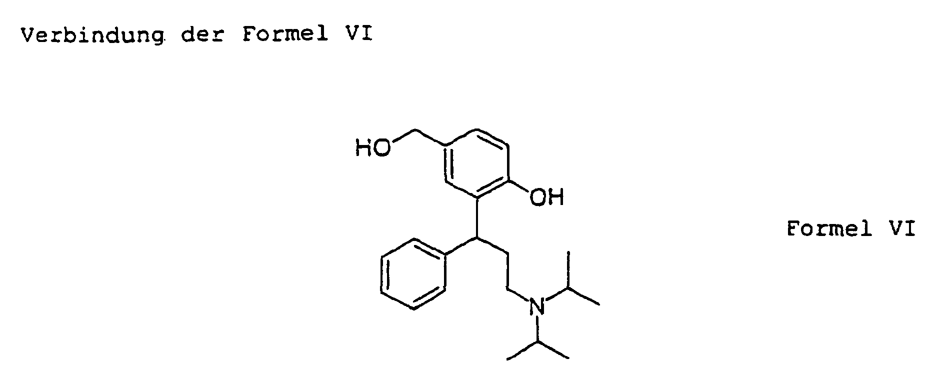 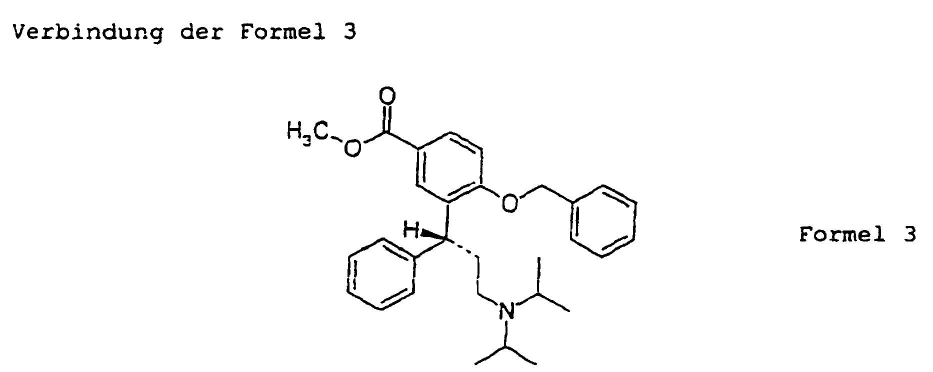 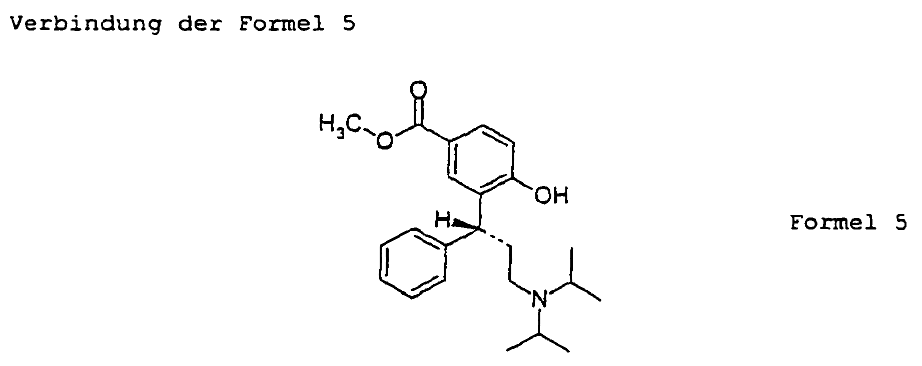 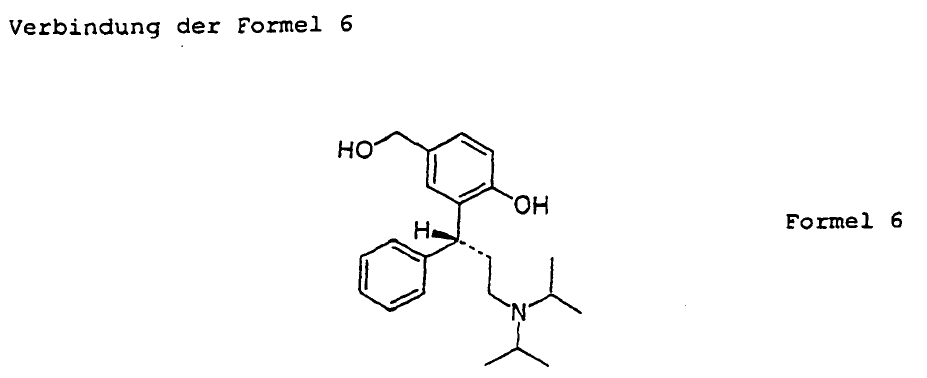 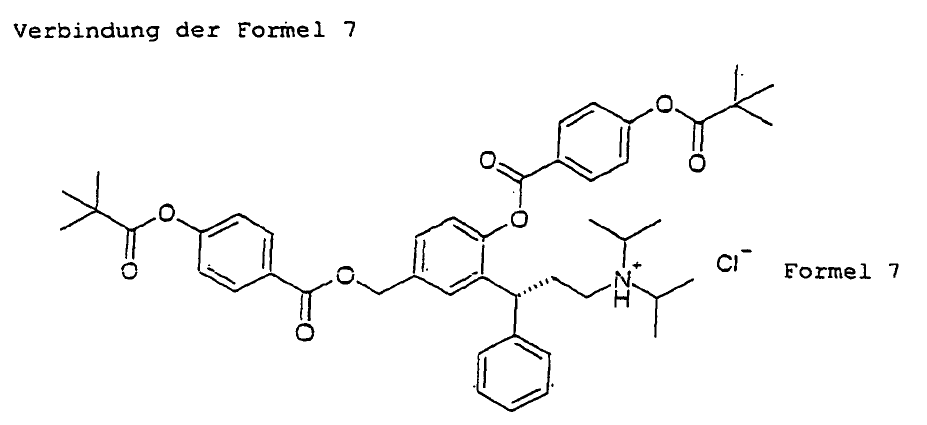 [(R)-3-(2-(1-[4-(2,2-Dimethyl-propanoyloxy)-phenyl]-methanoyloxy)-5-{1-[4-(2,2-dimethyl-propanoyloxy)-phenyl)-methanoyloxymethyl)-phenyl)-3-phenyl-propyl]-diisopropyl-ammoniumchlorid

Die vorgenannten Verbindungen III, V, VI, 3, 5, 6 und 7 eignen sich besonders zur Verwendung als jeweils hochreines, kristallines, stabiles Zwischenprodukt bei der Herstellung von pharmazeutisch nützlichen Verbindungen.

Besonders vorteilhaft eignen sich diese Verbindungen zur Verwendung als Zwischenprodukt bei der Herstellung von R-(+)-2-(3-Diisopropylamino-1-phenylpropyl)-4-hydroxymethylphenylisobuttersäureester Hydrogenfumarat und R-(+)-2-(3-Diisopropylamino-1-phenylpropyl)-4-hydroxymethylphenylisobuttersäureester Hydrochlorid Hydrat.

Schließlich kann das Verfahren besonders vorteilhaft ausgeführt werden, indem eine Verbindung der allgemeinen Formel 6 (siehe Reaktionsschema 1) mit einem Äquivalent Isobutyrylchlorid in Gegenwart von Triethylamin unter Verwendung eines der jeweiligen Lösungsmittel Ethylacetat, Dichlormethan, Tetrahydrofuran, Acetonitril oder Toluol regio- und chemoselektiv zu R-(+)-2-(3-Diisopropylamino-1-phenylpropyl)-4-hydroxymethylphenylisobuttersäureester umgesetzt wird.

Verfahrensgemäß eignet sich besonders vorteilhaft *R*-(+)-2-(3-Diisopropylarnino-1-phenylpropyl)-4-hydroxymethylphenylisobuttersäureester zur Umsetzung mit Fumarsäure oder Salzsäure unter Bildung des jeweiligen Salzes.

Die nachfolgenden Ausführungsbeispiele erläutern die Erfindung.

### Experimentelles

### I. Allgemeines

Alle Verbindungen wurden vollständig durch ¹H und ¹³C NMR-Spektroskopie charakterisiert (Bruker DPX 200). Die angeführten chemischen Verschiebungen in den ¹³C-NMR-Spektren (50 MHz, ppm Werte aufgeführt) beziehen sich auf die Lösungsmittelresonanzen von CDCl₃ (77.10 ppm). ¹H NMR Daten (CDCl₃; 200 MHz, ppm) beziehen sich auf internes Tetramethylsilan).

Dünnschichtchromatographie (DC, R_{f} angegeben) wurde durchgeführt auf 5x10 cm E. Merck Kieselgelfolien (60F254), die Flecken wurden visualisiert durch Fluoreszenzlöschung oder Ansprühen mit alkalischer Kaliumpermanganatlösung.

Laufmittelsysteme waren: (1), n-Hexan / Aceton / Triethylamin (70/20/10, v/v-%); (2), Toluol / Aceton / Methanol / Essigsäure (70/5/20/5, v/v-%).

Die optischen Drehungen wurden bei einer Wellenlänge von 589.3 nm (Natrium D-Linie) vermessen, bei Raumtemperatur unter Verwendung des Lösungsmittels Ethanol (Gerät: Perkin Elmer Polarimeter Type 241), Schmelzpunkte (Schmp., in °C) sind unkorrigiert und wurden am Gerät Mettler FP 1 bestimmt, bzw. Differentialthermoanalyse (DSC) am Perkin Elmer Modell DSC7, Auswertungssoftware "Pyris".

UV/VIS-Messungen wurden am Spektrophotometer Modell Lambda 7 (Perkin-Elmer) bei einer Schichtdicke von 1 cm durchgeführt. Angegeben ist die spezifische Absorption einer 1-%igen Lösung (A^{1 %}_{1 cm}).

IR-Spektren wurden an einem Perkin-Elmer FTIR Spektrometer Serie 1610 aufgezeichnet (Auflösung 4 cm⁻¹).

Gaschromatographie-Massenspektrometrie (GC-MS, m/z-Werte und relative Intensität bezogen auf das Basision (%)) wurde mit einem Finnigan TSQ 700 Triple Mass Spectrometer im positiv (P-CI) oder negativ (N-CI) chemische Ionisationsmeßbetrieb mit Methan oder Ammoniak als Reaktantgas bzw. über Elektronenstoßionisation aufgenommen. Hydroxyverbindungen wurden als Trimethylsilylether-Derivate vermessen.

Gekoppelte Flüssigkeitschromatographie-Massenspektrometrie (LC-MS): Waters Integrety System, Thermabeam Mass Detector (EI, 70 eV), m/z-Werte und relative Intensität (%) werden über einen Massenbereich von 50-500 a.m.u. angegeben.

### II. Ausführungsbeispiele

Die in Klammern gesetzten arabischen Zahlen (3), (4), (5), (6) beziehen sich auf die identischen Bezeichnungen im Reaktionsschema 1.

### 1. Darstellung von R-(-)-4-Benzyloxy-3-(3-diisopropylamino-1-phenyl-propyl)-benzoesäuremethylester (3)

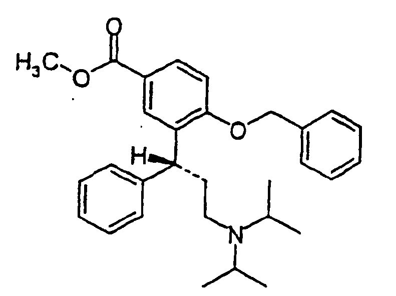

Eine Lösung von R-(-)-4-Benzyloxy-3-(3-diisopropylamino-1-phenyl-propyl)-benzoesäure Hydrochlorid (2.30 kg, 4.77 Mol) in 26.4 Liter Methanol und 0.25 Liter konzentrierter Schwefelsäure wird 16 Stunden unter Rückfluß erhitzt. Anschließend wird ein Drittel des Lösungsmittels abdestilliert, abgekühlt und unter Rühren mit 5 kg Eis und 2.5 Liter 25%-iger wässriger Kaliumcarbonatlösung versetzt. Der Ansatz wird erst mit 15 Liter, dann nochmals mit 5 Liter Dichlormethan extrahiert. Die organischen Phasen werden vereinigt und am Rotationsverdampfer zur Trockene eingeengt. Man erhält 1.99 kg (90.7 % der Theorie) hellgelbes Öl in ca. 90 % Reinheit (DC, NMR).
DC(1):0.58 ¹³C-NMR (CDCl₃): 20.55, 20.65, 36.83, 41.84, 43.83, 51.82, 70.12, 111.09, 122.46, 125.28, 127.49, 128.02, 128.35, 128.50, 129.22, 129.49, 133.20, 136.39, 144.51, 159.87, 167.09.

### Umkristallisation

69.0 g öliges Rohprodukt werden in 150 ml siedendem Methanol gelöst. Nach dem Zusatz von 15 ml destilliertem Wasser wird bei 0°C belassen, wobei sich farblose Kristalle abscheiden. Diese werden abfiltriert, mit wenig kaltem Methanol gewaschen und im Vakuum getrocknet. Ausbeute: 41.8 g (60.6 % der Theorie) farblose Kristalle, Schmp. 89.8 °C; [I]_{D}²⁰ = - 30.7 (c = 1.0, Ethanol).

### 2. Darstellung von R-(+)-[4-Benzyloxy-3-(3-diisopropylamino-1-phenyl-propyl)-phenyl)-methanol (4)

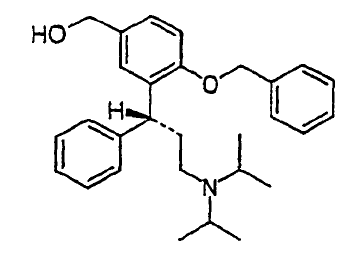

Rohprodukt (3) (28 g) wird in 230 ml absolutem Diethylether gelöst und unter Rühren in eine Suspension von 1.8 g Lithiumaluminiumhydrid in Diethylether (140 ml) getropft. Nach 18 Stunden Rühren bei Raumtemperatur werden tropfenweise 4.7 ml Wasser zugesetzt. Die organische Phase wird abgetrennt, mit wasserfreiem Natriumsulfat getrocknet, filtriert und am Rotationsverdampfer zur Trockene eingeengt. Man erhält 26 g (98.9 % der Theorie) *R*-(+) -[4-Benzyloxy-3-(3-diisopropylamino-1-phenyl-propyl)-phenyl]-methanol (4) als farbloses Öl. DC (2): 0.32; [I]_{D}²⁰ = + 6.3 (c = 1.0, Ethanol).
¹³C-NMR (CDCl₃): 20.53, 20.61, 36.87, 41.65, 44.14, 48.82, 65.12, 70.09, 111.80, 125.77, 125.97, 126.94, 127.55, 128.08, 128.37, 128.44, 133.27, 134.05, 134.27, 137.21, 144.84.

### 3. Darstellung von R-(-)-3-(3-Diisopropylamino-phenyl-propyl)-4-hydroxybenzoesäuremethylester (5)

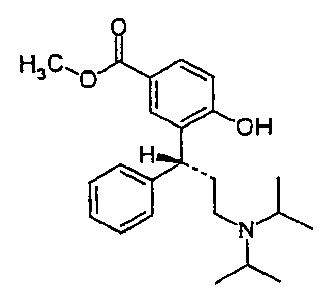

Zu einer gerührten Suspension von 5 g Raney-Nickel (mit Wasser, dann mit Methanol gewaschen) in 200 ml Methanol werden 10 g (21.8 mmol) R-(-)-4-Benzyloxy-3-(3-diisopropylamino-1-phenyl-propyl)-benzoesäuremethylester (3) zugesetzt. Nach kurzem Erwärmen, um alles (3) vollständig zu lösen, wird die Apparatur unter eine Atmosphäre von Wasserstoffgas gesetzt. Nach drei Stunden Rühren bei Normaldruck und Raumtemperatur zeigt die Dünnschichtchromatographie vollständige Umsetzung. Der Ansatz wird mit Stickstoffgas gespült und nach Zusatz von etwas Aktivkohle filtriert. Nach dem Einengen der methanolischen Lösung am Rotationsverdampfer verbleiben 6.0 g (75 % der Theorie) *R*-(-)-3-(3-Diisopropylaminophenyl-propyl)-4-hydroxy-benzoesäuremethylester (5) in Form farbloser Kristalle in einer Reinheit von 99.6 % (HPLC).

Schmp. 143.7 °C; DSC 144.7°C
[I]_{D}²⁰ = - 26. 6 (c = 0. 93, Ethanol).
¹³C-NMR (CDCl₃): 18.74, 19.21, 19.62, 33.12, 39.68, 42.36, 48.64, 51.42, 117.99, 120.32, 126.23, 127.81, 128.85, 129.39, 130.26, 132.21, 144.06, 162.43, 167.35.

### 4. Darstellung von R-(+)-2-(3-Diisopropylamino-1-phenylpropyl)-4-hydroxymethylphenol (6)

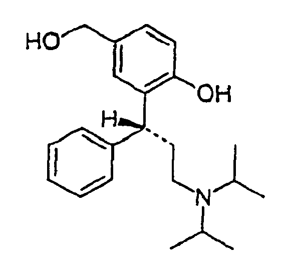

a) Ausgehend von der Zwischenstufe (4), R-(+)=[4-Benzyloxy-3-(3-diisopropylamino-1-phenyl-propyl)-phenyl)-methanol
   *R*-(+)-(4-Benzyloxy-3-(3-diisopropylamino-1-phenyl-propyl)-phenyl]-methanol (19.7 g, 45.7 mmol) werden in 220 ml Methanol gelöst und mit Raney-Nickel (5 g) versetzt. Die Apparatur wird mit Wasserstoffgas gespült und der Ansatz zwei Tage bei Raumtemperatur gerührt. Nach dem Zusatz von weiteren 5 g Raney-Nickel wird zwei weitere Tage bei Raumtemperatur unter Wasserstoffgasatmosphäre gerührt, vom Katalysator abfiltriert und das Filtrat am Rotationsverdampfer zur Trockene eingeengt. Derölige, blaßgelbe Rückstand wird in 100 ml Diethylether gelöst, zweimal mit je 100 ml Wasser gewaschen, über Natriumsulfat getrocknet, filtriert und zur Trockene eingeengt. Man erhält 14.1 g (90.4%der Theorie) *R*-(+)-2-(3-Diisopropylamino-1-phenylpropyl)-4-hydroxymethylphenol in Form eines cremefarbenen, amorphen Festkörpers. Umkristallisation siehe unten c).
b) Ausgehend von der Zwischenstufe (5); R-(-)-3-(3-Diiscpropylamino-phenyl-propyl)-4-hydroxy-benzoesäuremethylester
   Eine Lösung von 370 mg (1.0 mmol) R-(-)-3-(3-Diisopropylamino-phenyl-propyl)-4-hydroxy-benzoesäuremethylester in 20 ml wasserfreiem Tetrahydrofuran wird langsam und bei Raumtemperatur zu einer gerührten Mischung von trockenem Tetrahydrofuran (10 ml) und einer 1 M Lösung von Lithiumaluminiumhydrid in Tetrahydrofuran (3 ml) (unter Stickstoffschutzgasatmosphäre) getropft. Überschüssiges Hydrid wird durch tropfenweisen Zusatz einer gesättigten Natriumcarbonatlösung zersetzt. Nach dem Abtrennen der organischen Phase wird diese am Rotationsverdampfer eingeengt und anschließend im Hochvakuum getrocknet. Es werden 274 mg (74 % der Theorie) blaßgelbes Öl erhalten, das sich langsam zu einer amorphen Masse verfestigt.
c) Umkristallisation:
   Rohprodukt 6 (1.0 g) wird in Ethylacetat gelöst und am Rotationsverdampfer erneut eingeengt. Das so von
Fremdlösemitteln (Diethylether bzw. Tetrahydrofuran, s.o.) befreite Diol wird unter leichtem Erwärmen mit 1.5 ml Ethylacetat versetzt. Man rührt, bis eine klare Lösung entstanden ist, läßt auf Raumtemperatur abkühlen und setzt einige Impfkristalle zu. Letztere werden gewonnen, indem rohes 6 über HPLC gereinigt wird, die Hauptfraktion aufgefangen wird, eingeengt und der Rückstand mehrere Stunden im Hochvakuum getrocknet wird. Nachdem deutliche Kristallisation eingetreten ist, beläßt man bei - 10 °C. Die Kristalle werden noch in der Kälte abgesaugt und im Vakuum getrocknet. Man erhält farblose Kristalle in 84 % Ausbeute.

Schmp. 102.3 °C
DC (1) : 0.57
[I]_{D}²⁰ = + 21.3 (c = 1.0, Ethanol).
¹³C-NMR (CDCl₃): 19.58, 19.96, 33.30, 39.52, 42.10, 48.00, 65.40, 118.58, 126.31, 126.57, 127.16, 127.54, 128.57, 132.63, 132.83, 144.55, 155.52.

### 5. Darstellung von R-(+)-2-(3-Diisopropylamino-1-phenylpropyl)-4-hydroxymethylphenylisobuttersäureester (1)

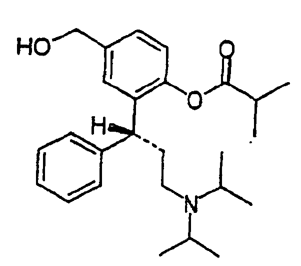

Eine Lösung von R-(+)-2-(3-Diisopropylamino-1-phenylpropyl)-4-hydroxymethylphenol (6) (65.0 g, 190.3 mmol) und Triethylamin (20.4 g, 201.7 mmol) in 750 ml Dichlormethan wird unter Rühren und Kühlung (-5 °C) mit einer Lösung von Isobuttersäurechlorid (23.4 g, 201.7 mmol) in 250 ml Dichlormethan versetzt. Nach der Zugabe wird noch 15 Min. bei 0 °C, dann 30 Min. bei Raumtemperatur gerührt und nacheinander mit Wasser (250 ml) und 5%-iger wässriger Natriumhydrogencarbonat-Lösung gewaschen. Die organische Phase wird abgetrennt und am Rotationsverdampfer zur Trockene eingeengt. Der Ester R-(+)-2-(3-Diisopropylamino-1-phenylpropyl)-4-hydroxymethylphenylisobuttersäureester wird als farbloses, viskoses Öl erhalten; Ausbeute: 77.1 g (98.4 % der Theorie).

DC (1) : 0.26; [I]_{D}²² = + 2.7 (c = 1.0, Ethanol).

¹³C-NMR (CDCl₃):19.01, 19.95, 20.59, 21.12, 34.28, 36.89, 41.88, 42.32, 43.90, 48.78, 64.68, 122.57, 125.59, 126.16, 126.86, 127.96, 128.54, 136.88, 138.82, 143.92, 147.90, 175.96.

### 6. Darstellung von R-(+)-2-(3-Diisopropylamino-1-phenylpropyl)-4-hydroxymethylphenylisobuttersäureester Hydrogenfumarat

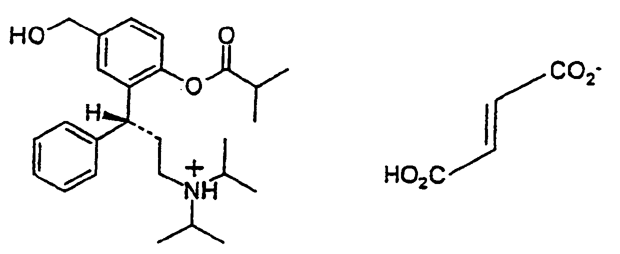

Eine Lösung von 41.87 g (102 mmol) R-(+)-2-(3-Diisopropylamino-1-phenylpropyl) -4-hydroxymethylphenylisobuttersäureester in 90 ml 2-Butanon wird unter Erwärmen mit Fumarsäure (11.81 g, 102 mmol) versetzt. Nach dem Lösen der Säure wird langsam unter Rühren Cyclohexan (20-30 ml) bis zum Einsetzen einer Trübung zugesetzt. Man beläßt den farblosen, homogenen Ansatz zunächst 18 Stunden bei Raumtemperatur, dann mehrere Stunden bei 0 °C. Die ausgefallenen farblosen Kristalle werden abgesaugt, mit wenig Cyclohexan/2-Butanon (90:10, Vol.-%) gewaschen und im Vakuum bei 30 °C getrocknet. Man erhält 44.6 g (83.1 % der Theorie) des Hydrogenfumarat-Salzes des R-(+)-2-(3-Diisopropylamino-1-phenylpropyl)-4-hydroxymethylphenylisobuttersäureester in Form farbloser Plättchen.

Schmp. 98.8 °C, eine zweite Kristallisation aus dem gleichen Lösungsmittelgemisch ergibt das Produkt mit einem Schmp. von 103 °C.

[I]_{D}²⁰ = + 6.0 (c = 1.0, Ethanol).

Elementaranalyse: Berechnet für C₃₀H₄₁NO₇ (Molgewicht 527.66) C 68.29 %, H 7.83 %, N 2.65 %, O 21.2 %; gefunden C 68.29 %, H 7.90 %, N 2.72 %, O 21.0 %.

UV/VIS bei Σ in nm (A ^{1%}_{1 cm}): 191 (1306), 193 (1305), 200 (1143), 220 (456).

IR: 3380, 2978, 2939, 2878, 2692, 2514, 1756, 1702, 1680, 1618, 1496, 1468, 1226, 1040, 1019, 806,

¹H-NMR (CDCl₃): 1.198, 1.285, 1.287 (CH₃); 2.541 (CHC=0); 3.589 (NCH); 4.585 (CH₂OH); 6.832 (=CH, Fumarat); 6.84-7.62 (Aryl, = CH).

¹³C-NMR (CDCl₃): 17.79, 18.95, 19.16 (CH₃); 31.63 (CHCH₂); 34.09 (CH-C=O); 41.87 (CHCH₂); 45.83 (NCH₂); 54.29 (NCH); 63.78 (OCH₂);122.23,126.48,126.77,127.56, 140.46,140.52,142.35, 147.54 (Aryl CH); 135.54 (=CH, Fumarat); 170.48 (C=O, Fumarat); 175.62 (i-Pr-C=O).

MS im Direkteinlaß, m/z (%): 411 (1), 396 (9), 380 (1), 223 (2), 165 (2), 114 (100), 98 (4), 91 (3), 84 (3), 72 (10), 56 (7).

### 7. Darstellung von R-(+)-2-(3-Diisopropylamino-1-phenylpropyl)-4-hydroxymethylphenylisobuttersäureester Hydrochlorid Hydrat

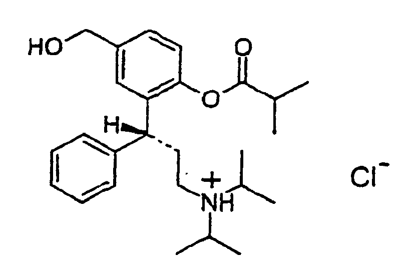

Eine Lösung von R-(+)-2-(3-Diisopropylamino-1-phenylpropyl)-4-hydroxymethylphenylisobuttersäureester (8.54 g, 25.0 mmol) in 50 ml Dichlormethan wird bei 0°C langsam in eine gerührte Lösung von Isobuttersäurechlorid (2.66 g, 25.0 mmol) in 100 ml Dichlormethan getropft. Nach einer Stunde wird die Kühlung entfernt und noch eine Stunde bei Raumtemperatur nachgerührt. Nach dem Abziehen der flüchtigen Bestandteile im Vakuum am Rotationsverdampfer hinterbleibt ein farbloser, amorph-fester Schaum. Dieser Rückstand wird in Aceton (17 ml) gelöst, mit 0.45 bis 0.50 g Wasser und Diethylether versetzt (ca.20 - 25 ml) bis deutliche Trübung eintritt. Nach kurzer Behandlung mit Ultraschall tritt spontane Kristallisation ein und es werden unter Rühren langsam weitere 80 ml Diethylether zugetropft. Die ausgefallenen farblosen Kristalle werden abgesaugt und über Nacht im Vakuum über Phosphorpentoxid getrocknet. Man erhält 10.5 g (93.7 % der Theorie) farblos kristallines R-(+)-2-(3-Diisopropylamino-1-phenylpropyl)-4-hydroxymethylphenylisobuttersäureester Hydrochlorid Hydrat in 97.0 % Reinheit (HPLC).

Schmp. 97.1 °C.

[I]_{D}²⁰ = + 4.3 (c = 1.03, Ethanol)

¹³C-NMR (CDCl₃): 16.94, 17.35, 18.24, 18.40, 18.87, 19.05, 31.20, 33.99, 41.64, 45.41, 54.18, 54.42, 63.83, 122.25, 126.50, 126.70, 126.96, 127.34, 128.60, 133.80, 140.55, 142.17, 147.68, 175.79.

### 8. Phenolische Monoester

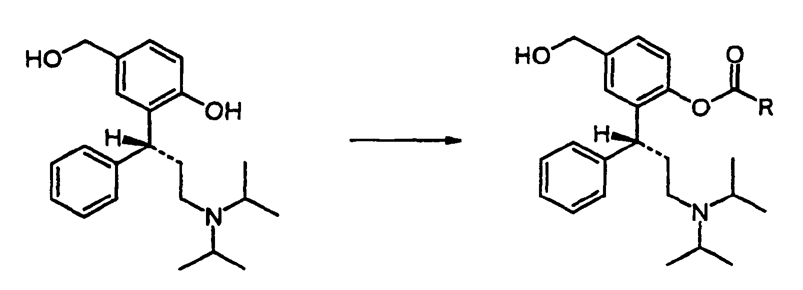

### Allgemeine Arbeitsvorschrift zur Hersteilung von phenolischen Monoestern

In eine Lösung von 120.3 mg (0.352 mmol) R-(+)-2-(3-Diisopropylamino-1-phenylpropyl)-4-hydroxyphenol (6) in 5 ml Dichlormethan wird unter Rühren bei 0 °C eine Lösung von Säurechlorid (0.352 mmol) in 2 ml Dichlormethan eingetropft. Anschließend wird mit Triethylamin-Dichlormethan (49.1 µl/0.353 mmol-2 ml) versetzt. Nach 18 Stunden bei Raumtemperatur zeigt die Dünnschichtchromatographie vollständige Umsetzung. Der Ansatz wird nacheinander mit 5 ml Wasser, wässriger 0.1 N-Salzsäure, 5 ml 5%-iger wässriger Natriumhydrogencarbonatlösung, 5 ml Wasser gewaschen, über Natriumsulfat getrocknet, und nach der Filtration zur Trockene eingeengt. Anschließend wird im Hochvakuum bis zur Gewichtskonstanz getrocknet.

Nach diesem Verfahren wurden folgende Verbindungen beispielhaft hergestellt:

### R = CH₂CH(CH₃)₂

### R-(+)-3-Methylbuttersäure-2-(3-diisopropylamino-1-phenylpropyl)-4-hydroxymethylphenyl-ester

Farbloses Öl in 70% Ausbeute und >95% Reinheit (NMR).
¹³C-NMR (CDCl₃): 20.45, 20.59, 22.54, 25.70, 36,74, 42.18, 43.27, 43.96, 48.90, 64.67, 122.66, 125.60, 126.20, 126.79, 127.95, 128.37, 136.83, 138.86, 143.83, 147.82, 171.37.
DC (1): 0.76.

### R = CH₂C (CH₃)₃

### R-(+)-3,3-Dimethylbuttersäure-2-(3-diisopropylamino-1-phenylpropyl)-4-hydroxymethylphenyl-ester, freie Base.

Farbloses Öl in 69.7% Ausbeute und >95% Reinheit (NMR).
¹³C-NMR (CDCl₃): 20.40, 20.53, 29.73, 30.99, 36.62, 42.17, 44.01, 47.60, 49.01, 64.65, 122.64, 125.60, 126.20, 126.80, 127.96, 128.36, 136.85, 138.90, 143.80, 147.82, 170.55.
DC (1): 0.75.

### R = (CH₃)₃C

### R-(+)-Pivalinsäure-2-(3-diisopropylamino-1-phenyl-propyl)-4-hydroxymethylphenyl-ester Hydrochlorid.

Farblose Kristalle, Schmp. 165-6 °C.
¹³C-NMR (DMSO-d₆ =39.7 ppm): 16.52, 16.68, 17.98, 18.11, 26.87, 31.46, 41.71, 45.33, 53.89, 53.98, 62.65, 122.61, 122.97, 125.94, 126.09, 126.57, 126.75, 127.87, 128.58, 131.80, 134.94, 141.02, 142.69,' 147.17, 155.32,' 163.92, 176.21.

### R = C-C₃H₅

### R-(+)-Cyclopropancarbonsäure-2-(3-diisopropylamino-1-phenylpropyl)-4-hydroxymethylphenyl-ester Hydrochlorid.

Farblose wachsartige Masse.
¹³C-NMR (DMSO-d₆ =39.7 ppm): 173.02, 172.49, 172.37, 153.10, 147.12, 142.72, 142.03, 140.78, 136.60, 134.79, 134.35, 129.55, 129.13, 128.80, 128.67, 127.87, 126.96, 126.74, 125.94, 125.84, 124.37, 123.71, 122.80, 62.64, 53.92, 45.34, 41.65, 31.44, 18.05, 16.66, 12.84, 9.58, 9.28, 8.49, 7.89.

### R = c-C₄H₇

### R-(+)-Cyclobutancarbonsäure-2-(3-diisopropylamino-1-phenylpropyl)-4-hydroxymethylphenyl-ester Hydrochlorid

Farblose wachsartige Masse.
¹³C-NMR (DMSO-d₆ =39.7 ppm): 173.53, 147.12, 142.81, 140.74, 134.77, 128.65, 127.81, 126.74, 125.99, 125.87, 122.75, 62.63, 53.92, 45.34, 41.42, 37.38, 31.54, 25.04, 24.92, 18.03, 16.68, 16.61.

### R = c-C₅H₉

### R-(+)-Cyclopentancarbonsäure-2-(3-diisopropylamino-1-phenylpropyl)-4-hydroxymethylphenyl-ester Hydrochlorid

Farblose wachsartige Masse.
¹³C-NMR (DMSO-d₆ =39.7 ppm): 174.80, 147.22, 142.86, 140.76, 134.72, 128.66, 127.80, 126.73, 126.04, 125.88, 122.71, 62.62, 53.94, 45.37, 43.24, 41.39, 31.54, 29.78, 29.59, 25.64, 25.59, 18.07, 16.64.

### R = c-C₆H₁₁

### R-(+)-Cyclohexancarbonsäure-2-(3-diisopropylamino-1-phenylpropyl)-4-hydroxymethylphenyl-ester Hydrochlorid

Farblose wachsartige Masse.
¹³C-NMR(DMSO-d₆ =39.7 ppm):
174.08, 147.15, 142.85, 140.77, 134.78, 128.66, 127.77, 126.74, 126.06, 125.87, 122.69, 62.61, 53.91, 45.36, 42.26, 41.24, 31.53, 28.74, 28.62, 25.48, 25.04, 24.98, 18.05, 16.67, 16.60.

### R = 4- (C₂H₅CO₂)-C₆H₄

### R-(+)-4-(Ethylcarbonyloxy)-benzoesäuresäure-2-(3-diisopropylamino-1-phenyl-propyl)-4-hydroxymethylphenyl-ester Hydrochlorid

Farblose Kristalle, Schmp. 195-8 °C.
¹H-NMR (DMSO-d₆): 9.87 (s, 1H mit D₂O austauschbar, NH), 8.19-8.12 (m, 2H, Phenyl-H), 7.55 (d, J = 1.0 Hz, 1H, Phenyl-H3), 7.41-7.13 (m, 9H, Phenyl-H), 5.28 (br s, 1H mit D₂O austauschbar, OH), 4.53 (s, 2H, CH₂), 4.23 (t, J = 7.6 Hz, 1H, CH), 3.61-3.50 (m, 2H, 2 × CH(CH₃)₂), 2.97-2.74 (m, 2H, CH₂), 2.67 (q, J = 7.4 Hz, 2H, CH₂), 2.56-2.43 (m, 2H, CH₂), 1.23-1.13 (m, 15H, 2 × CH(CH₃)₂, CH₃).

### R = 4-(i-C₃H₇CO₂)-C₆H₄

### R-(+)-4-(Isopropylcarbonyloxy)-benzoesäuresäure-2-(3-diisopropylamino-1-phenyl-propyl)-4-hydroxymethylphenylester Hydrochlorid

Farblose Kristalle, Schmp. 202-4 °C.
¹H-NMR (DMSO-d₆) : 9.73 (s, 1H mit D₂O austauschbar, NH), 8.19-8.12 (m, 2H, Phenyl-H), 7.55 (d, J = 1.4 Hz, 1H, Phenyl-H3), 7.42-7.14 (m, 9H, Phenyl-H), 5.27 (br s, 1H mit D₂O austauschbar, OH), 4.53 (s, 2H, CH₂), 4.23 (t, J = 7.5 Hz, 1H, CH), 3.61-3.50 (m, 2H, 2 × CH(CH₃)₂), 2.99-2.78 (m, 3H, CH₂, CH(CH₃)₂), 2.54-2.47 (m, 2H, CH₂), 1.29-1.13 (m, 18H, 3 × CH(CH₃)₂).

### R = 4-(t-C₄H₉CO₂)-C₆H₄

### R-(+)-4-(t-Butylcarbonyloxy)-benzoesäuresäure-2-(3-diisopropylamino-1-phenyl-propyl)-4-hydroxymethylphenylester, freie Base.

Farbloses Öl.
¹H-NMR (DMSO-d₆): 8.19-8.12 (m, 2H, Phenyl-H), 7.45-7.33 (m, 3H, Phenyl-H), 7.25-7.09 (m, 7H, Phenyl-H), 5.20 (t, J = 5.6 Hz, 1H, OH), 4.50 (d, J = 5.6 Hz, 2H, CH₂), 4.20 (t, J = 7.5 Hz, 1H, CH), 2.95-2.80 (m, 2H, 2 × CH(CH₃)₂), 2.38-2.25 (m, 2H, CH₂), 2.09-2.03 (m, 2H, CH₂), 1.33 (s, 9H, (CH₃)₃), 0.82-0.76 (m, 12H, 2 × CH (CH₃)₂).

Hydrochlorid: farblose Kristalle, Schmp. 165-6 °C.
¹H-NMR (CDCl₃): 8.22-8.16 (m, 2H, Phenyl-H), 8.02 (d, J = 1.8 Hz, 1H, Phenyl-H), 7.27-7.02 (m, 9H, Phenyl-H), 4.83-4.60 ('m', 2H, CH₂), 4.01-3.94 (m, 1H, CH), 3.66-3.54 (m, 2H), 3.18-2.80 (m, 3H), 2.53-2.44 (m, 1H) (2 × CH₂, 2 × CH(CH₃)₂), 1.43-1.25 (m, 21H, (CH₃)₃, 2 × CH(CH₃)₂).

### R = 4- (c-C₃H₅CO₂) -C₆H₄

### R-(+)-4-(Cyclopropylcarbonyloxy)-benzoesäuresäure-2-(3-diisopropylamino-1-phenyl-propyl)-4-hydroxymethylphenyl-ester. Hydrochlorid

Farblose Kristalle, Schmp. 208-213 °C.
¹H-NMR (DMSO-d₆): 9.04 (s, 1H mit D₂O austauschbar, NH), 8.15-8.09 (m, 2H, Phenyl-H), 7.53 ('d', 1H, Phenyl-H3), 7.42-7.13 (m, 9H, Phenyl-H), 5.25 (br s, 1H mit D₂O austauschbar, OH), 4.52 (s, 2H, CH2), 4.23 (t, J = 7.5 Hz, 1H, CH), 3.62-3.53 (m, 2H, 2 × CH(CH3)2), 3.05-2.70 (m, 2H, CH2), 2.51-2.37 (m, 2H, CH2), 2.01-1.89 (m, in, Cyclopropyl-CH), 1.20-1.05 (m, 16H, 2 × CH(CH3)2, 2 × Cyclopropyl-CH2).

¹³C-NMR(DMSO-d₆ = 39.7 ppm): 172.71, 163.93, 154.92, 147.16, 142.69, 141.03, 134.97, 131.76, 128.60, 127.86, 126.76, 126.56, 126.06, 125.94, 122.95, 122.65, 62.65, 54.00, 53.89, 45.33, 41.63, 31.49, 18.10, 17.98, 16.69, 16.51, 12.86, 9.52.

### R = 4-(c-C₄H₇CO₂)-C₆H₄

### R-(+)-4-(Cyclobutylcarbonyloxy)-benzoesäuresäure-2-(3-diisopropylamino-1-phenyl-propyl)-4-hydroxymethylphenylester Hydrochlorid

Farblose Kristalle, Schmp. 201-6 °C.
¹H-NMR (DMSO-d₆): 9.50 (s, 1H mit D₂O austauschbar, NH), 8.17-8.12 (m, 2H, Phenyl-H), 7.54 (d, J = 1.4 Hz, 1H, Phenyl-H3), 7.42-7.14 (m, 9H, Phenyl-H), 5.25 (br s, 1H mit D₂O austauschbar, OH), 4.52 (s, 2H, CH₂), 4.23 (t, J = 7.5 Hz, 1H, CH), 3.62-3.47 (m, 3H, Cyclobutyl-CH), 2 × CH(CH₃)₂), 3.00-2.70 (m, 2H, CH₂), 2.51-2.26 (m, 6H, CH₂, 2 × Cyclobutyl-CH₂), 2.10-1.85 (m, 2H, Cyclobutyl-CH₂), 1.22-1.12 (m, 12H, 2 × CH(CH₃)₂).

### R = 4- (c-C₆H₁₁CO₂)-C₆H₄

### R-(+)-4-(Cyclohexylcarbonyloxy)-benzoesäuresäure-2-(3-diisopropylamino-1-phenyl-propyl)-4-hydroxymethylphenyl-ester Hydrochlorid

Farblose Kristalle, Schmp. 212-217 °C.
¹H-NMR (DMSO-d₆): 9.34 (s, 1H, *mit D₂O austauschbar,* NH), 8.16-8.12 (m, 2H, Phenyl-H), 7.54 (d, J = 1.4 Hz, 1H, Phenyl-H3), 7.39-7.14 (m, 9H, Phenyl-H), 5.26 ('t', 1H, *mit D₂O austauschbar,* OH), 4.53 (d, J = 4.2 Hz, 2H, CH₂), 4.22 (t, J = 7.5 Hz, 1H, CH), 3.62-3.48 (m, 2H, 2 × CH(CH₃)₂), 3.00-2.60 (m, 3H, Cyclohexyl-CH), CH₂), 2.51-2.40 (m, 2H, CH₂), 2.07-1.98 (m, 2H, Cyclohexyl-CH₂), 1.80-1.1 (m, 20H, 4 × Cyclohexyl-CH₂), 2 × CH (CH₃)₂)

### 9. Identische Diester

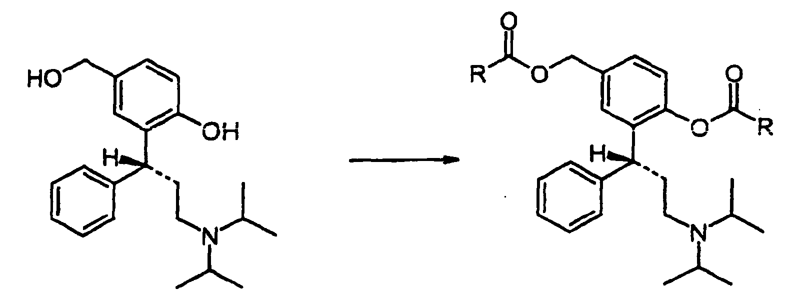

### Allgemeine Arbeitsvorschrift zur Herstellung von identischen Diestern

In eine Lösung von 7.30 g (21.4 mmol) R-(+)-2-(3-Diisopropylamino-1-phenylpropyl)-4-hydroxyphenol (6) in 100 ml Dichlormethan wird unter Rühren bei 0 °C eine Lösung von Säurechlorid (49.2 mmol) in 50 ml Dichlormethan eingetropft. Anschließend wird mit Triethylamin-Dichlormethan (6.86 ml/49.2 mmol-50 ml) versetzt. Nach 1-3 Stunden bei Raumtemperatur zeigt die Dünnschichtchromatographie vollständige Umsetzung. Der Ansatz wird nacheinander mit jeweils 100 ml Wasser, wässriger 0.1 N-Salzsäure, 5 ml 5%-iger wässriger Natriumhydrogencarbonatlösung, 5 ml Wasser gewaschen, über Natriumsulfat getrocknet, und nach der Filtration zur Trockene eingeengt. Anschließend wird im Hochvakuum bis zur Gewichtskonstanz getrocknet.

Nach diesem Verfahren wurden folgende Verbindungen beispielhaft hergestellt:

### R = Methyl

### R-(-)-Essigsäuresäure-2-(3-diisopropylamino-1-phenyl-propyl)-4-acetoxymethyl-phenyl-ester, freie Base

Blaßgelbes Öl, Reinheit (HPLC): 95.2%.
¹³C-NMR (CDCl₃): 20.36, 20.69, 20.94, 20.99, 36.41, 42.27, 43.69, 48.79, 65.89, 122.89, 126.28, 127.17, 127.92, 128.36, 133.69, 136.95, 143.61, 148.46, 168.97, 170.76.
LC-MS: 425 (15%, M⁺), 410 (97%), 382 (4%), 308 (3%), 266 (7%), 223 (27%), 195 (13%), 165 (8%), 114 (100%).
[α]_{D}²⁰ = -33.1 (c = 1, CH₃CN).
DC (1): 0.79.

### R = Cyclohexyl

### R-Cyclohexancarbonsäure-2-(3-diisopropylamino-1-phenylpropyl)-4-cyclohexylcarbonyloxymethyl-phenyl-ester

Blaßgelbes Öl, Reinheit (NMR): >95%.
¹³C-NMR (CDCl₃): 20.30, 25.17, 25.58, 25.73, 28.97, 29.12, 41.70, 43.15, 44.03, 48.64, 65.37, 122.67, 125.88, 126.24, 127.06, 127.31, 127.90, 128.37, 134,03, 136.85, 143.55, 148.33, 174.20, 175.72.
DC (1): 0.96.

### R = Isopropyl

### R-Isobuttersäure-2-(3-diisopropylamino-1-phenyl-propyl)-4-isobutyryloxymethyl-phenyl-ester

Freie Base: blaßgelbes Öl, Reinheit (HPLC): 95.6%.
¹³C-NMR (CDCl₃): 18.96, 19.08, 20.59, 33.98, 34.20, 36.86, 41.72, 43.72, 48.72, 65.58, 122.65, 126.19, 126.73, 127.91, 128.11, 128.36, 133.91, 136.96, 143.81, 148.41, 175.15, 176.77.
DC (1): 0.74.

Hydrogenfumarat-Salz: farbloser Sirup, 94.4% HPLC-Reinheit.
¹³C-NMR (CDCl₃) : 17.89,
18.07, 18.94, 18.97, 19.07, 31.22, 33:93, 34.13, 41.78, 45.62, 53.93, 65.33, 122.93, 126.82, 127.45, 127.53, 127.91, 128.75, 134. 74, 135.29, 135.42, 142.04, 148.44, 170.24, 175.71, 176.79.

### R = 4-(t-C₄H₉CO₂)-C₆H₄

### R-4-(t-Butylcarbonyloxy)-benzoesäure-2-(3-diisopropylamino-1-phenyl-propyl)-4-(4-t-butylcarbonyloxymethyl-benzoesäure)-phenyl-ester Hydrochlorid

Farblose Kristalle, Schmp. 10.5-7 °C.
¹³C-NMR (DMSO-d₆): 16.49, 16.71, 17.97, 18.06, 26.84, 31.36, 38.45, 41.70, 45.24, 53.79, 53.96, 55.09, 66.11, 122.47, 122.62, 123.59, 126.42, 126.83, 127.21, 127.70, 127.88, 128.02, 128.62, 131.17, 131.86, 134.48, 135.64, 142.52, 148.35, 154.86, 155.39, 163.80, 165.09, 176.14, 176.19.

### 10. Gemischte Diester

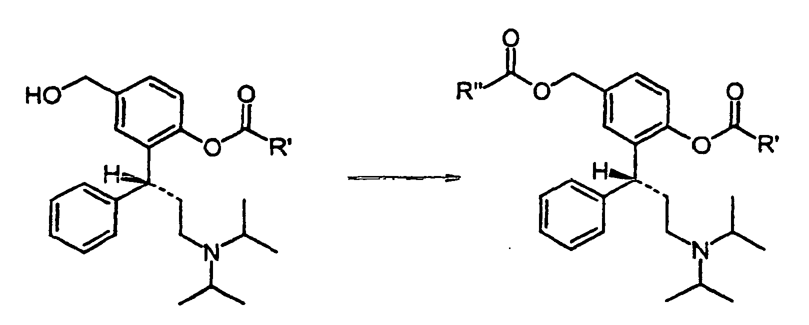

R' ist ungleich R"

### Allgemeine Arbeitsvorschrift zur Herstellung von gemischten Diestern

In eine Lösung von 5.30 mmol phenolischen Monoester der allgemeinen Formel A in 40 ml Dichlormethan wird unter Rühren bei 0 °C eine Lösung von Säurechlorid (5.83 mmol) in 15 ml Dichlormethan eingetropft. Anschließend wird mit Triethylamin-Dichlormethan (0.589g/5.82 mmol-15 ml) versetzt. Nach 18 Stunden bei Raumtemperatur zeigt die Dünnschichtchromatographie vollständige Umsetzung. Der Ansatz wird nacheinander mit jeweils 50 ml Wasser, wässriger 0.1 N-Salzsäure, 5 ml 5%iger wässriger Natriumhydrogencarbonatlösung, 5 ml Wasser gewaschen, über Natriumsulfat getrocknet, und nach der Filtration zur Trockene eingeengt. Anschließend wird im Hochvakuum bis zur Gewichtskonstanz getrocknet.

Nach diesem Verfahren wurden folgendes Beispiel hergestellt:

### R' = CH(CH₃)₂

### R" = CH₃

### R-Isobuttersäure-2-(3-diisopropylamino-1-phenyl-propyl)-4-acetoxymethyl-phenyl-ester

Farbloses Öl.
DC (1): 0.56
¹³C-NMR (CDCl₃) : 19.12, 20.65, 21.05, 34.24, 37.02, 41.79, 43.79, 48.72, 65.98, 122.75, 125.98, 126.22, 127.94, 128.39, 128.84, 133.55, 137.04, 143.84, 148.58, 170.84, 175.18.

Hydrochlorid: farblose Kristalle
¹³C-NMR (CDCl₃): 16.89, 17.04, 18.31, 18.92, 20.95, 31.49, 34.07, 41,64, 46.17, 54.55, 65.49, 122.91, 126.61, 126.93, 127.48, 127.83, 128.74, 134.50, 134.88, 141.61, 148.44, 170.67, 175.63.
[α]_{D}²⁰ = +14.6 (c = 1, CHCl₃).

## Patentansprüche

1. Verbindungen der allgemeinen Formel I 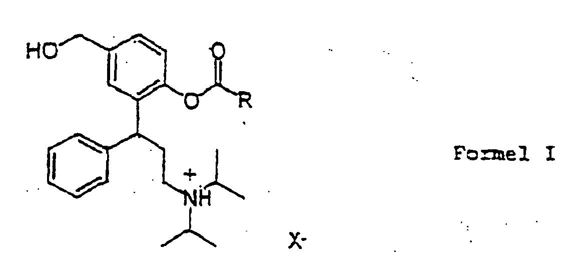 worin R für C₁-C₆-alkyl, C₃-C₁₀-cycloalkyl, substituiertes oder unsubstituiertes Phenyl steht und X⁻ der Säurerest einer physiologisch verträglichen anorganischen oder organischen Säure ist, mit der Maßgabe, dass die Verbindung nicht R-(+)-Isobuttersäure-2-(3-diisopropylamino-1-phenylpropyl)-4-hydroxymethylphenylester Hydrochlorid ist.

2. Verbindungen nach Anspruch 1, **dadurch gekennzeichnet, daß** X⁻ jeweils ein Säurerest der Salzsäure, Bromwesserstottsäure, Phosphorsäure, Schwetelsäure, Salpetersäure, Essigsäure, Propionsäure, Palmitinsäure, Stearinsäure, Meleinsäure, Fumarsäure, Oxalsäure. Bernsteinsäure DL-Äpfelsäure, L-(-)-Äpfelsäure, D-(+)-Äpleisäure, DL-Weinsäure, L-(+)-Neinsäure, D-(-)-Weinsäure, Citronensäure, L-Asparaginsäure, L-(+)-Ascorbinsäure, D-(+)-Glucuronsäure, 2-Oxopropionsäure (Brenztraubensäure), Furan-2-carbonsäure (Brenzschleimsäure), Benzoesäure, 4-Hydroxybenzoesäure Salicylsäure, Vanillinsäure, 4-Hydroxyzimtsäure, Gallussäure, Hippursäure (N-Benzoyl-glycin), Acetursäure (N-Acetylglycin), Phioretinsäure (3-(4-Hydroxyphenyl)-propionsäure), Phthalsäure, Methansulphonsäure oder der Orotsäure ist.

3. Verbindungen nach Ansprüchen 1 und 2, **dadurch gekennzeichnet**, das sie die aligemeine Formel 2 besitzen, 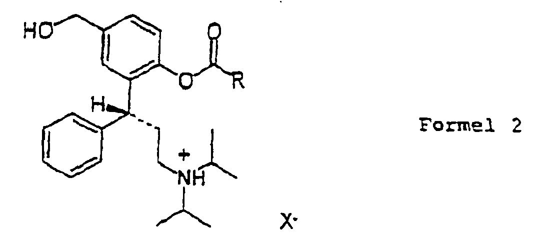 worin R für C₁-C₆-alkyl, C₃-C₁₀-cycloalkyl, substituiertes oder unsubstituiertes Phenyl steht und X⁻ der Säurerest einer physiologisch verträglichen anorganischen oder organischen Säure ist.

4. Verbindungen nach Anspruch 3, **dadurch gekennzeichnet, daß** X⁻ jeweils ein Säurerest der Salzsäure, Bromwesserstoffsäure, Phosphorsäure, Schwetelsäure, Salpetersäure, Essigsäure, Propionsäure, Palmitinsäure, Stearinsäure, Maleinsäure, Fumarsäure, Oxatsäure, Bernsteinsäure, DL-Äpfeisäure, L-(-)-Äpfelsäure, D-(+)-Äpfelsäure, DL-Weinsäure, L-(+)-Welnsäure, D-(-)-Weinsäure, Citronensäure, L-Asparaginsäure, L-(+)-Ascorbinsäure, D-(+)-Glucuronsäure, 2-Gxoprooionsäure (Brenmaubensäure), Furen-2-carbonsäure (Brenzschteirnsäure);
Benzoesäure, 4-Hydroxybenzoesäure, Salicylsäure, Vanillinsäure, 4-Hydroxyzimtsäure, Gallussäure, Hippursäure (N-Benzoyl-glycin), Acetursäure (N-Acetyiglycin), Phloretinsäure (3-(4-Hyaroxypnenyl)-propionsäure), Phthalsäure, Methansulphonsäure oder der Orotsäure ist.

5. Verbindungen nach Ansprüchen 3 und 4, **dadurch gekennzeichnet daß** sie *R*-(+)-2-(3-(Diisopropylainino-1-phenylpropyl)-4-hydroxymethyl-phenylisobuttersäureesterhydrogentumarat, R-(+)-2-(3-(Diisopropylamino-1-phenylpropy)-4-hydroxymethylphenylisobuttersäureester-hydrochioridhydrat sind.

6. Verbindungen nach Ansprüchen 3 und 4, **dadurch gekennzeichnet, daß** R für Cyclopropyl, Cyclobutyl, Cyclopentyl, Cyclohexyl, 4-(1-Cyclopropyl-methanoyloxy)-phenyl, 4-(1-Cyclobutyl-methanoyloxy) -Phenyl, 4-(1-Cyclohexyl-methanoyloxy)-phenyl oder 4-(22-Dimethyl-propanoyloxy)-phenyl steht und X⁻ Chlorid bedeutet.

7. Verbindungen nach Ansprüchen 1 bis 6 in Form eines Schtüttgutes.

8. Verfahren zur Herstellung von Verbindungen der allgemeinen Formel I 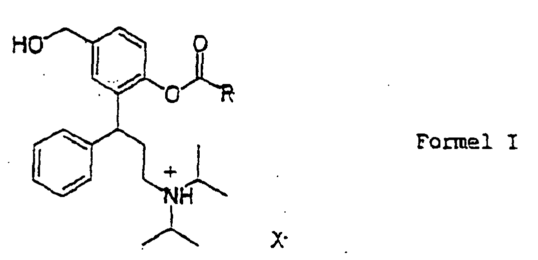 worin R für C₁-C₆-alkyl, C₃-C₁₀-cycloalkyl, substituiertes oder unsubstituiertes Phenyl steht und X⁻ für den Säurerest einer physiologisch verträglichen anorganischen oder organischen Säure steht, **dadurch gekennzeichnet, daß**
a)-eine Verbindung der Formel III 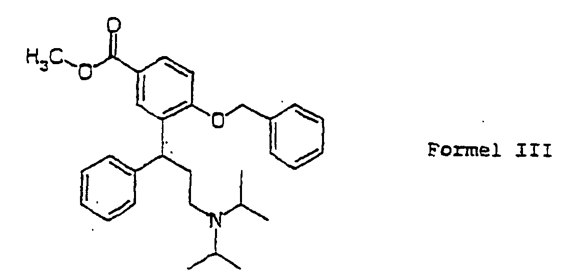 mit einem Hydrierungsmittel zur Bildung einer Verbindung der Formel V 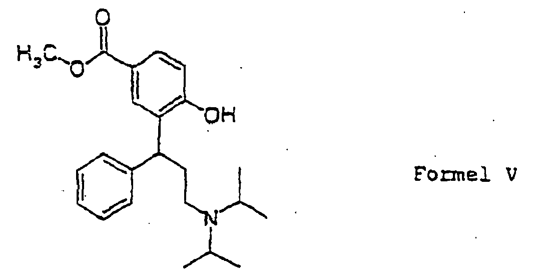 gespalten wird, worauf
b) die so erhaltene Verbindung der Formel V mit einem Reduktionsmittel umgesetzt wird, um eine Verbindung der Formel VI 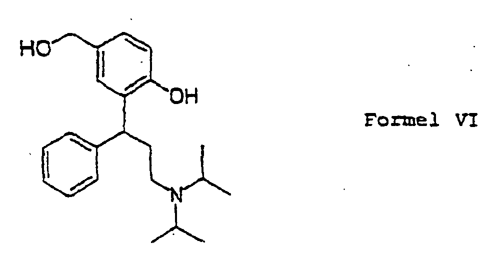 zu ergeben, die
c) mit einem Acylierungsmittel umgesetzt wird, um eine Verbindung der Formel A 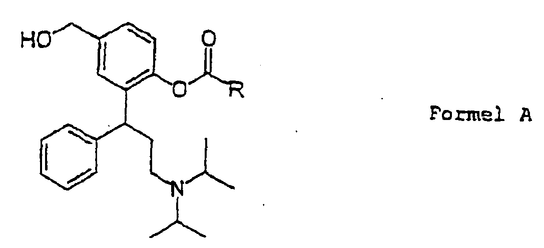 zu erhalten, worin R die oben genannte Bedeutung hat, die
d) mit einer physiologisch verträglichen anorganischen oder organischen Säure unter Bindung einer verbindung der Formel I 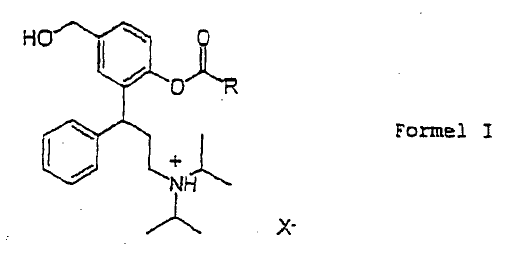 umgesetzt wird, worin R für C₁-C₆-alkyl, C₃-C₁₀-cycloalkyl, unsubstituiertes oder substituiertes Phenyl steht und X⁻ für den Säurerest einer physiologisch verträglichen anorganischen oder organischen Säure steht.

9. Verfahren nach Anspruch 8, **dadurch gekennzeichnet, daß** zur Herstellung der Verbindungen der allgemeinen Formel I die Säuren salzsäure, Bromwasserstoffsäure, Phosphorsäure, Schwefelsäure, Salpetersäure, Essigsäure, Propionsäure, Palmitinsäure, Stearinsäure, maleinsäure, Fumarsäure, Oxalsäure, Bernsteinsäure, DL-Äpfelsäure, L-(-) Äpfelsäure, D-(+)-Äpfelsäure, DL-Weinsäure, L-(+)-Weinsäure, D-(-)-Weinsäure, Citronensäure, L-Asparaginsäure, L-(+)-Ascorbinsäure, D-(+)-Glucuronsäure, 2-Oxopropionsäure (Brenztreubensäure), Furan-2-carbonsäure (Brenzschleimsäure) Benzoesäure, 4-Hydroxybenzoesäure, Sallcyisäure, Vanillinsäure, 4-Hydroxyzimtsäure, Gallussäure, Hippursäure (N-Benzoyl-glycin), Acetursäure (N-Acetylglycin), Phloretinsäure (3-(4-Hydroxyphenyl)-propionsäure), Phthalsäure, Methansulphonsäure oder Orotsäure verwendet werden.

10. Verfahren zur Herstellung von Verbindungen der allgemeinen Formel 2 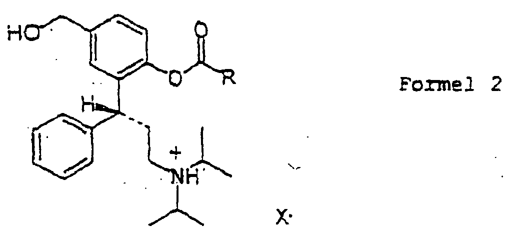 worin R für C₁-C₆-alkyl, C₃-C₁₀-cycloalkyl, substituiertes oder unsubstituiertes Phenyl steht und X⁻ für den Säurerest einer physiologisch verträglichen anorganischen oder organischen Säure steht, **dadurch gekennzeichnet, daß**
a) eine Verbindung der Formel 3 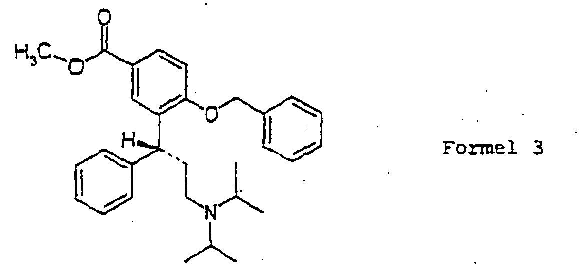 mit einem Hydrierungsmittel zur Bildung einer Verbindung der Formel 5 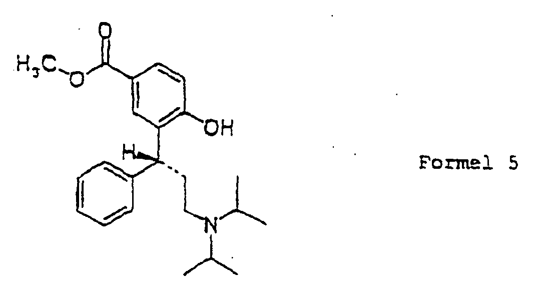 gespalten wird, worauf
b) die so erhaltene Verbindung der Formel 5 mit einem Reduktionsmittel umgesetzt wird, um eine Verbindung der Formel 6 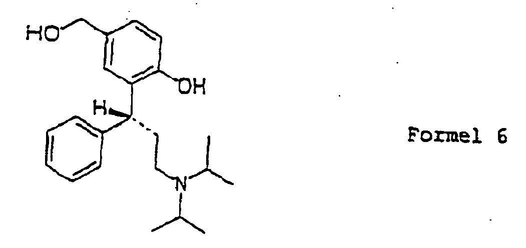 zu ergeben, die
c) mit einem Acylierungsmittel umgesetzt wird, um eine Verbindung der Formel 1 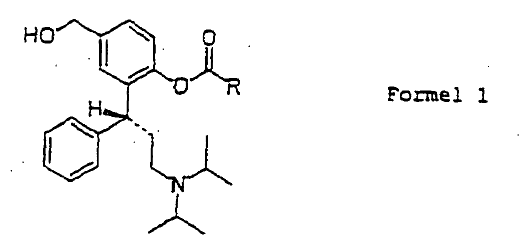 zu erhalten, worin R die oben genannte Bedeutung hat, die
d) mit einer physiologisch verträglichen anorganischen oder organischen Säure unter Bildung einer Verbindung der Formel 2 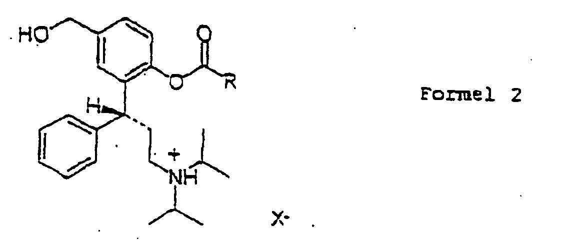 umgesetzt wird, worin R für C₁-C₆alkyl, C₃-C₁₀-cycloalkyl, unsubstituiertes oder substituiertes Phenyl steht und X- für den Säurerest einer physiologisch verträglichen anorganischen oder organischen Säure sieht.

11. Verfahren nach Anspruch 10. **dadurch gekennzeichnet, daß** zur Herstellung der Verbindungen der allgemeinen Formel 2 die Säuren Salzsäure. Bromwasserstofisäure, Phosphorsäure, Schwefelsäure, Salpetersäure, Essigsäure, Propionsäure, Palmitinsäure, Stearinsäure, Maleinsäure, Fumarsäure, Oxalsäure, Bernsteinsäure, DL-Äptelsäure, L-(-)-Apfeisäure; D- (+) -Äpfeisäure, DL-Weinsäure, L-(+)-Weinsäure, D-(-)-Weinsäure, Citronensäure, L-Asparaginsäure. L-(+)-Ascorbinsäure, D-(+)-Glucuronäure, 2-Oxopropionsäure (Brenztraubensäure), Furan-2-carbonsäure (Brenzschleimsäure) Benzoesäure, 4-Hydroxybenzoesäure, Salicylsäure, Vanillinsäure, 4-Hydroxyzimtsäure, Gallussäure, Hippursäure (N-Benzoyl-glycin), Acetursäure (N-Acetylglycin), Phioretinsäure (3-(4-Hydroxyphenyl)-propionsäure), Phthalsäure, Methansulphonsäure oder Orotsäure verwendet werden.

12. Verfahren nach Ansprüchen 8 bis 11, **dadurch gekennzeichnet, daß** ais Hydrierungsmittel vorzugsweise Raney-Nickel/H₂ in Methanol als Lösungsmittel verwendet wird.

13. Verfahren nach Ansprüchen 8 bis 11, **dadurch gekennzeichnet**, das als Reduktionsmitel NaBH₄/EtOH, vorzugsweise LiAlH₄/THF verwendet werden.

14. Verfahren nach Ansprüchen 8 bis 11, **dadurch gekennzeichnet, daß** als Acylierungsmittel Isobutyrylchlorid und als Base Triethylamin verwendet werden.

15. Verfahren nach Ansprüchen 10 bis 14, **dadurch gekennzeichnet, daß** eine Verbindung der Formel 6 mit einem Äquivalent isobutyrylchlorid in Gegenwart von Triethylamin unter Verwendung eines der jeweiligen Lösungsmittel Ethylacetat, Dichlormethan, Tetrahydrofuran, Acetonitril oder Toluol regio- und chemoselektiv zu R-(+)-2-(3-Diisopropylamino-1-phenylpropyl)-4-hydroxymethylphenylisobuttersäureester umgesetzt wird.

16. Verfahren nach Ansprüchen 10 bis 15, **dadurch gekennzeichnet, daß** R-(+)-2-(3-Diisopropylamino-1-phenylpropyl)-4-hydroxymethylphenylisobuttersäureester und Fumarsäure oder Salzsäure unter Bildung des jeweiligen Salzes umgesetzt werden.

17. Verfahren nach Ansprüchen 10 bis 13 zur Herstellung von R-(+)-2-(3-Diisopropylamino-1-phenyl-propyl)-4-hydroxymethylphenylisobuttersäureester Hydrochlorid Hydrat, **dadurch gekennzeichnet, daß** die phenolische Veresterung von R-(+)-2-(3-Diisopropylamino-1-phenylpropyl)-4-hydroxymethylphenol (6) ohne Zusatz einer externen Base durchgeführt wird, indem Lösungen von (6) in Lösungen von Isobuttersäurechlorid, die mindestens 1 Moläquivalent Wasser enthalten, zugetropft werden, um direkt ein entsprechendes stabiles, hydrathaltiges Hydrochlorid zu erhalten.

## Claims

1. Compounds of general formula I 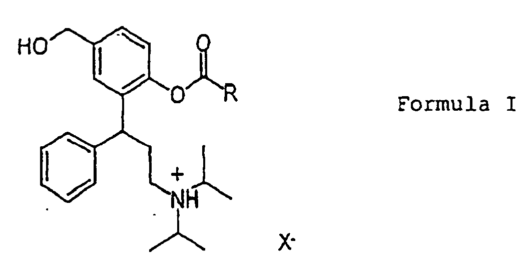 in which R denotes C₁-C₆-alkyl, C₃-C₁₀-cycloalkyl, substituted or unsubstituted phenyl and X- is the acid residue of a physiologically compatible inorganic or organic acid, with the proviso that the compound is not R-(+)-isobutyric acid 2-(3-diisopropylamino-1-phenylpropyl)-4- hydroxymethylphenyl ester hydrochloride,

2. Compounds in accordance with claim 1, **characterised in that** X- in each case is an acid residue of hydrochloric acid, hydrobromic acid, phosphoric acid, sulfuric acid, nitric acid, acetic acid, propionic acid, palmitic acid, stearic acid, maleic acid, fumaric acid, oxalic acid, succinic acid, DL-malic acid, L-(-)-malic acid, D-(+)-malic acid, DL-tartaric acid, L-(+)-tartaric acid, D-(-)-tartaric acid, citric acid, L-aspartic acid, L-(+)-ascorbic acid, D-(+)-glucuronic acid, 2-oxopropionic acid (pyruvic acid), furan-2-carboxylic acid (mucic acid), benzoic acid, 4-hydroxy benzoic acid, salicyclic acid, vanillic acid, 4-hydroxy cinammic acid, gallic acid, hippuric acid (N-benzoyl glycine), aceturic acid (N-acetyl glycine), phloretinic acid (3-(4-hydroxyphenyl)propionic acid), phthalic acid, methane sulfonic acid or orotic acid.

3. Compounds in accordance with claims 1 and 2, **characterised in that** they have general formula 2 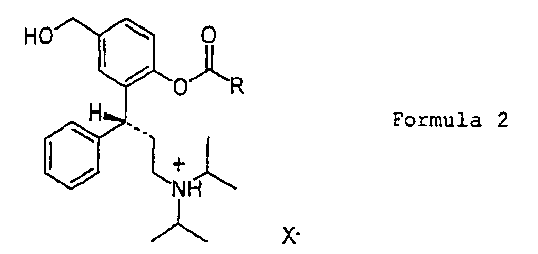 in which R denotes C₁-C₆-alkyl, C₃-C₁₀-cycloalkyl, substituted or unsubstituted phenyl and X- is the acid residue of a physiologically compatible inorganic or organic acid.

4. Compounds in accordance with claim 3, **characterised In that** X- in each case is an acid residue of hydrochloric acid, hydrobromic acid, phosphoric acid, sulfuric acid, nitric acid, acetic acid, propionic acid, palmitic acid, stearic acid, maleic acid, fumaric acid, oxalic acid, succinic acid, DL-malic acid, L-(-)-malic acid, D-(+)-malic acid, DL-tartaric acid, L-(+)-tartaric acid, D-(-)-tartaric acid, citric acid, L-aspartic acid, L-(+)-ascorbic acid, D-(+)-glucuronic acid, 2-oxopropionic acid (pyruvic acid), furan-2-arboxylic acid (mucic acid), benzoic acid, 4-hydroxy benzoic acid, salicyclic acid, vanillic acid, 4-hydroxy cinammic acid, gallic acid, hippuric acid (N-benzoyl glycine), aceturic acid (N-acetyl glycine), phloretinic acid (3-(4-hydroxyphenyl)propionic acid), phthalic acid, methane sulfonic acid or orotic acid.

5. Compounds In accordance with claims 3 and 4, **characterised in that** they are *R*-(+)-2-(3-(diisopropylamino-1-phenylpropyl)-4-hydroxymethylphenylisobutyrate hydrogen fumarate, R-(+)-2-(3-(diisopropylamino-1-phenylpropyl)-4-hydroxymethylphenylisobutyrate hydrochloride hydrate.

6. Compounds in accordance with claims 3 and 4, **characterised in that** R represents cyclopropyl, cyclobutyl, cyclopentyl, cyclohexyl, 4-(1-cyclopropyl-methanoyloxy)-phenyl, 4-(1-cyclobutyl-methanoyloxy)-phenyl, 4-(1-cyclohexyl-methanoyloxy)-phenyl or 4-(2,2-dimethyl-propanoyloxy)-phenyl and X- denotes chloride.

7. Compounds in accordance with claims 1 to 6 in the form of a bulk material.

8. Method for manufacturing compounds of general formula I 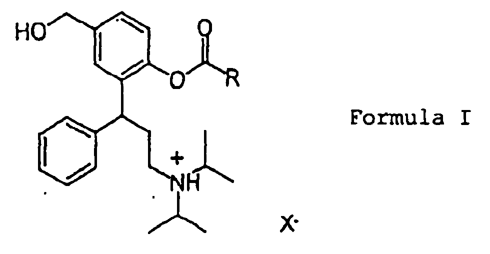 in which R denotes C₁-C₆-alkyl, C₃-C₁₀-cycloalkyl, substituted or unsubstituted phenyl and X- is the acid residue of a physiologically compatible inorganic or organic acid, **characterised in that**
a) a compound of formula III 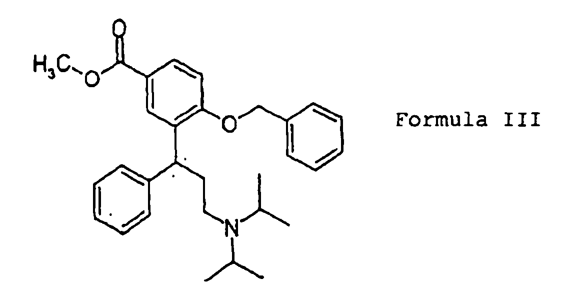 is cleaved with a hydrogenation agent to form a compound of Formula V 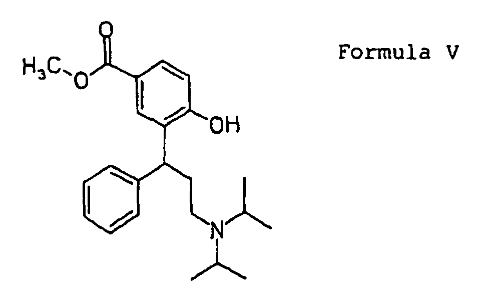 whereupon
b) the compound of formula V so obtained is reacted with a reducing agent, in order to give a compound of formula VI 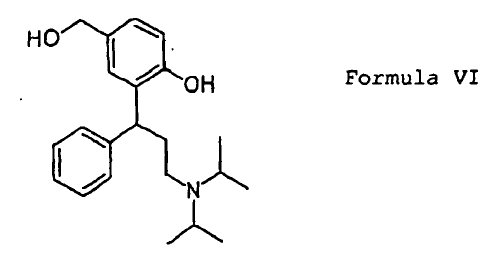 which
c) is reacted with an acylation agent, in order to obtain a compound of formula A 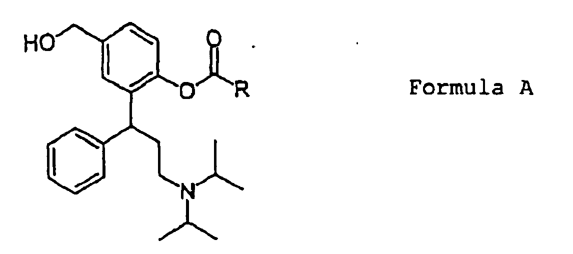 in which R has the significance stated above, which
d) is reacted with a physiologically compatible inorganic or organic acid to form a compound of formula I 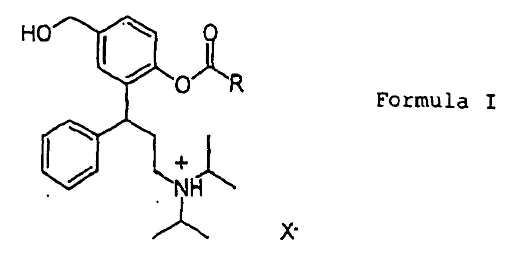 in which R denotes C₁-C₆-alkyl, C₃-C₁₀-cycloalkyl, unsubstituted or substituted phenyl and X- is the acid residue of a physiologically compatible inorganic or organic acid.

9. Method in accordance with claim 8, **characterised in that** for the manufacture of the compounds of general formula I hydrochloric acid, hydrobromic acid, phosphoric acid, sulfuric acid, nitric acid, acetic acid, propionic acid, palmitic acid, stearic acid, maleic acid, fumaric acid, oxalic acid, succinic acid, DL-malic acid, L-(-)-malic acid, D-(+)-malic acid, DL-tartaric acid, L-(+)-tartaric acid, D-(-)-tartaric acid, citric acid, L-aspartic acid, L-(+)-ascorbic acid, D-(+)-glucuronic acid, 2-oxopropionic acid (pyruvic acid), furan-2-carboxylic acid (mucic acid), benzoic acid, 4-hydroxy benzoic acid, salicyclic acid, vanillic acid, 4-hydroxy cinammic acid, gallic acid, hippuric acid (N-benzoyl glycine), aceturic acid (N-acetyl glycine), phloretinic acid (3-(4-hydroxyphenyl)propionic acid), phthalic acid, methane sulfonic acid or orotic acid are used.

10. Method for manufacturing compounds of general formula 2 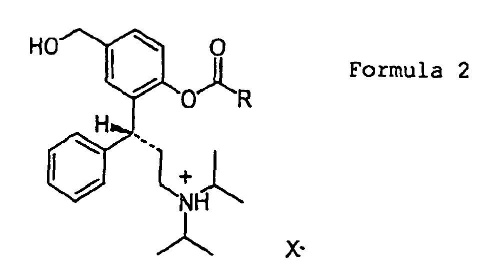 in which R denotes C₁-C₆-alkyl, C₃-C₁₀-cycloalkyl, substituted or unsubstituted phenyl and X- is the acid residue of a physiologically compatible inorganic or organic acid, **characterised in that**
a) a compound of the formula 3 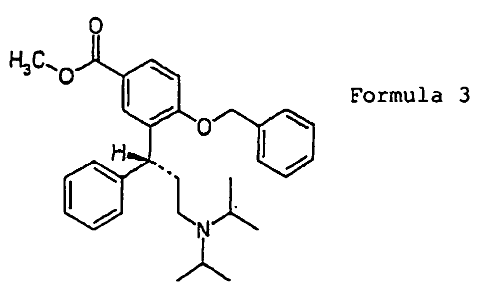 is cleaved with a hydrogenation agent to form a compound of formula 5 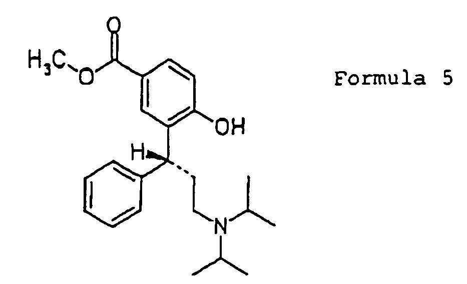 whereupon
b) the compound of formula 5 so obtained is reacted with a reducing agent, in order to give a compound of formula 6 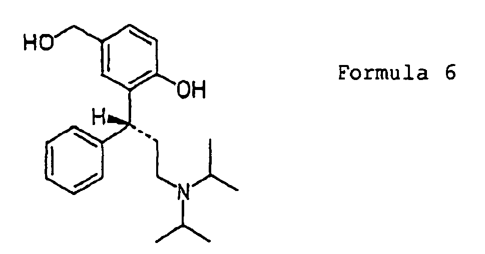 which
c) is reacted with an acylation agent, in order to obtain a compound of formula 1 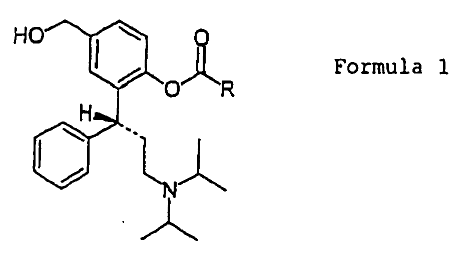 in which R has the significance stated above, which
d) is reacted with a physiologically compatible inorganic or organic acid to form a compound of formula 2 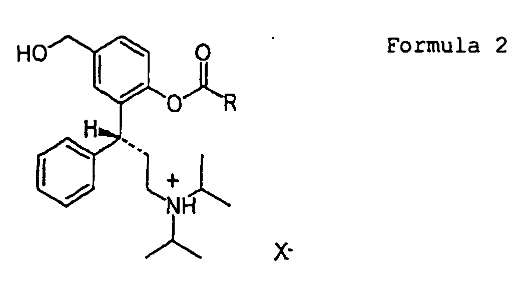 in which R denotes C₁-C₆-alkyl, C₃-C₁₀-cycloalkyl, unsubstituted or substituted phenyl and X- is the acid residue of a physiologically compatible inorganic or organic acid.

11. Method in accordance with claim 10, **characterised in that** for the manufacture of the compounds of general formula 2 hydrochloric acid, hydrobromic acid, phosphoric acid, sulfuric acid, nitric acid, acetic acid, propionic acid, palmitic acid, stearic acid, maleic acid, fumaric acid, oxalic acid, succinic acid, DL-malic acid, L-(-)-malic acid, D-(+)-malic acid, DL-tartaric acid, L-(+)-tartaric acid, D-(-)-tartaric acid, citric acid, L-aspartic acid, L-(+)-ascorbic acid, D-(+)-glucuronic acid, 2-oxopropionic acid (pyruvic acid), furan-2-carboxylic acid (mucic acid), benzoic acid, 4-hydroxy benzoic acid, salicyclic acid, vanillic acid, 4-hydroxy cinammic acid, gallic acid, hippuric acid (N-benzoyl glycine), aceturic acid (N-acetyl glycine), phloretinic acid (3-(4-hydroxyphenyl)propionic acid), phthalic acid, methane sulfonic acid or orotic acid are used.

12. Method in accordance with claims 8 to 11, **characterised in that** as the hydrogenation agent Raney nickel/H₂ in methanol as a solvent is preferably used.

13. Method in accordance with claims 8 to 11, **characterised in that** as the reducing agent NaBH₄/EtOH, preferably LiAIH₄/THF, is used.

14. Method in accordance with claims 8 to 11, **characterised in that** as the acylation agent isobutyryl chloride and as the base triethylamine are used.

15. Method in accordance with claims 10 to 14, **characterised in that** a compound of general formula 6 is reacted with an equivalent isobutyryl chloride in the presence of triethylamine using one of the respective solvents ethylacetate, dichloromethane, tetrahydrofurane, acetonitrile or toluene regio- and chemoselectively into R-(+)-2-(3-diisopropylamino-1-phenylpropyl)-4-hydroxymethylphenylisobutyrate.

16. Method in accordance with claims 10 to 15, **characterised in that** R-(+)-2-(3-diisopropylamino-1-phenylpropyl)-4-hydroxymethylphenylisobutyrate and fumaric acid or hydrochloric acid are reacted to form the corresponding salt.

17. Method in accordance with claims 10 to 13 for the manufacture of R-(+)-2-(3-diisopropylamino-1-phenylpropyl)-4-hydroxymethylphenylisobutyrate hydrochloride hydrate, **characterised in that** the phenolic esterification of R-(+)-2-(3-diisopropylamino-1-phenylpropyl)-4-hydroxymethylphenol (6) is carried out without the addition of an external base by dropping solutions of (6) into solutions of isobutyrate chloride, that contain at least 1 mole equivalent of water, in order to directly obtain a corresponding stable, water-containing hydrochloride.

## Revendications

1. Composés de la formule générale I 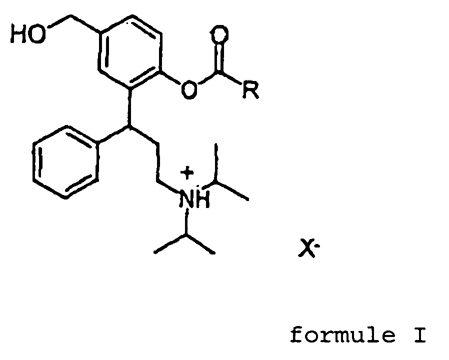 dans laquelle R représente un alkyle en C₁ à C₆, un cycloalkyle en C₃ à C₁₀, un phényle substitué ou non substitué et X⁻ le résidu acide d'un acide inorganique ou organique physiologiquement compatible, sous réserve que le composé ne soit pas le chlorhydrate de l'ester R-(+)-2-(3-diisopropylamino-1-phénylpropyl)-4-hydroxyméthylphénylique de l'acide isobutyrique.

2. Composés selon la revendication 1, **caractérisés en ce que** X⁻ est respectivement un résidu acide de l'acide chlorhydrique, de l'acide bromhydrique, de l'acide phosphorique, de l'acide sulfurique, de l'acide nitrique, de l'acide acétique, de l'acide propionique, de l'acide palmitique, de l'acide stéarique, de l'acide maléique, de l'acide fumarique, de l'acide oxalique, de l'acide succinique, de l'acide malique DL, de l'acide malique L (-), de l'acide malique D (+),de l'acide tartrique DL, de l'acide tartrique L (+),de l'acide tartrique D (-), de l'acide citrique, de l'acide aspartique L, de l'acide ascorbique L (+), de l'acide glucuronique D (+), de l'acide 2-oxopropionique (acide pyruvique), de l'acide furanne-2-carboxylique (acide mucique), de l'acide benzoïque, de l'acide 4-hydroxybenzoïque, de l'acide salicylique, de l'acide vanillique, de l'acide 4-hydroxycinnamique, de l'acide gallique, de l'acide hippurique (N-benzoyl-glycine), de l'acide acéturique (N-acétylglycine), de l'acide phlorétique (acide 3-(4-hydroxyphényl)propionique), de l'acide phtalique, de l'acide méthane sulfonique ou de l'acide orotique.

3. Composés selon les revendications 1 et 2, **caractérisés en ce qu'**ils possèdent la formule générale 2, 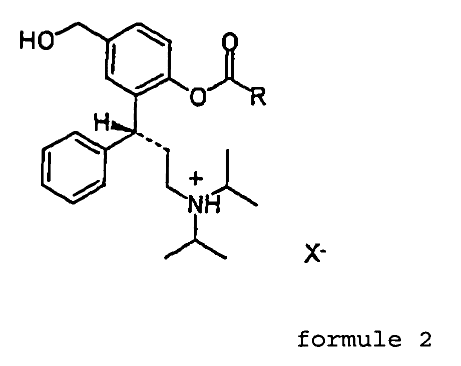 dans laquelle R représente un alkyle en C₁ à C₆, un cycloalkyle en C₃ à C₁₀, un phényle substitué ou non substitué et X⁻ est le résidu acide d'un acide inorganique ou organique physiologiquement compatible.

4. Composés selon la revendication 3, **caractérisés en ce que** X⁻ est respectivement un résidu acide de l'acide chlorhydrique, de l'acide bromhydrique, de l'acide phosphorique, de l'acide sulfurique, de l'acide nitrique, de l'acide acétique, de l'acide propionique, de l'acide palmitique, de l'acide stéarique, de l'acide maléique, de l'acide fumarique, de l'acide oxalique, de l'acide succinique, de l'acide malique DL, de l'acide malique L (-), de l'acide malique D (+),de l'acide tartrique DL, de l'acide tartrique L (+),de l'acide tartrique D (-), de l'acide citrique, de l'acide aspartique L, de l'acide ascorbique L (+), de l'acide glucuronique D (+), de l'acide 2-oxopropionique (acide pyruvique), de l'acide furanne-2-carboxylique (acide mucique), de l'acide benzoïque, de l'acide 4-hydroxybenzoïque, de l'acide salicylique, de l'acide vanillique, de l'acide 4-hydroxycinnamique, de l'acide gallique, de l'acide hippurique (N-benzoyl-glycine), de l'acide acéturique (N-acétylglycine), de l'acide phlorétique (acide 3-(4-hydroxyphényl)propionique), de l'acide phtalique, de l'acide méthane sulfonique ou de l'acide orotique.

5. Composés selon les revendications 3 et 4, **caractérisés en ce qu'**ils sont des hydrogénofumarates de l'ester isobutyrique de R-(+)-2-(3-(diisopropylamino-1-phénylpropyl)-4-hydroxyméthyl-phényle, des hydrochlorures hydratés de l'ester isobutyrique de R-(+)-2-(3-(diisopropylamino-1-phénylpropyl)-4-hydroxyméthyl-phényle.

6. Composés selon les revendications 3 et 4, **caractérisés en ce que** R représente un radical cyclopropyle, cyclobutyle, cyclopentyle, cyclohexyle, 4-(1-cyclopropyl-méthanoyloxy)-phényle, 4-(1-cyclobutyl-méthanoyloxy)-phényle, 4-(1-cyclohexyl-méthanoyloxy)-phényle, ou 4-(2,2-diméthylpropanoyloxy)-phényle, et X⁻ signifie chlorure.

7. Composés selon les revendications 1 à 6, en forme de produit en vrac.

8. Procédé pour la fabrication de composés de la formule générale I 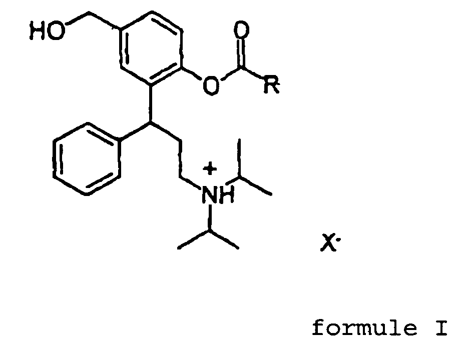 dans laquelle R représente un alkyle en C₁ à C₆, un cycloalkyle en C₃ à C₁₀, un phényle substitué ou non substitué et X⁻ est le résidu acide d'un acide inorganique ou organique physiologiquement compatible, **caractérisé en ce que**
a) un composé de la formule III 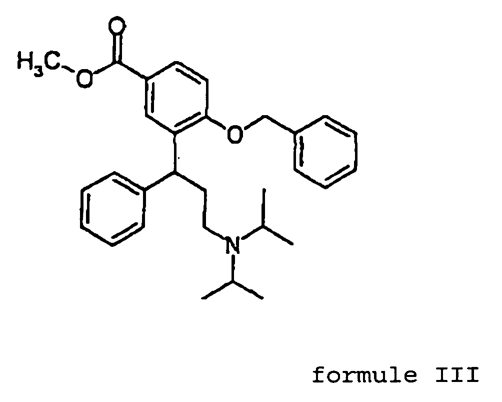 est décomposé avec un hydrogénant pour former un composé de la formule V 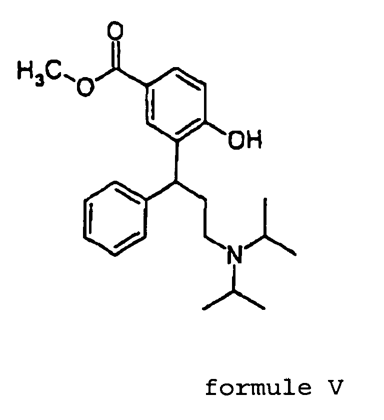 après quoi
b) le composé de la formule V ainsi obtenu est transformé avec un agent réducteur pour donner un composé de la formule VI 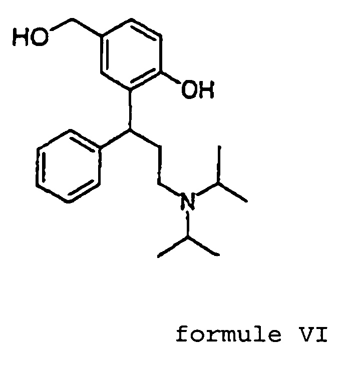 qui
c) est transformé avec un agent d'acylation pour donner un composé de la formule A 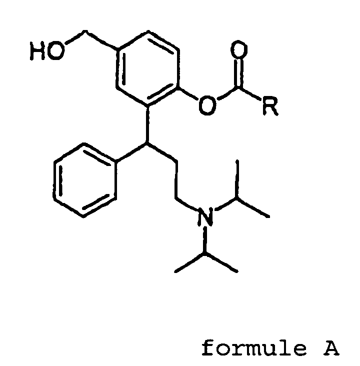 dans laquelle R a la signification définie ci-dessus, composé qui
d) est transformé avec un acide inorganique ou organique physiologiquement compatible moyennant la formation d'un composé de la formule 1 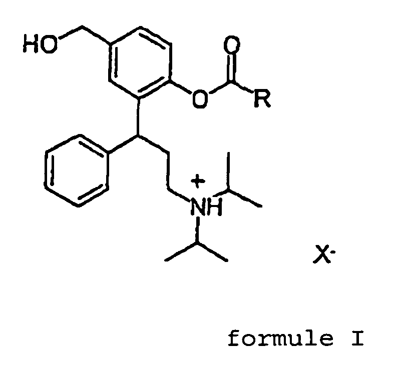 dans laquelle R représente un alkyle en C₁ à C₆, un cycloalkyle en C₃ à C₁₀, un phényle substitué ou non substitué et X⁻ est le résidu acide d'un acide inorganique ou organique physiologiquement compatible.

9. Procédé selon la revendication 8, **caractérisé en ce que**, pour la fabrication des composés de la formule générale I, sont utilisés les acides : chlorhydrique, bromhydrique,
phosphorique, sulfurique, nitrique, acétique, propionique, palmitique, stéarique, maléique, fumarique, oxalique, succinique, malique DL, malique L (-), malique D (+), tartrique DL, tartrique L (+), tartrique D (-), citrique, aspartique L, ascorbique L (+), glucuronique D (+), 2-oxopropionique (acide pyruvique), furanne-2-carboxylique (acide mucique), benzoïque, 4-hydroxybenzoïque, salicylique, vanillique, 4-hydroxycinnamique, gallique, hippurique (N-benzoyl-glycine), acéturique (N-acétylglycine), phlorétique (acide 3-(4-hydroxyphényl)propionique), phtalique, méthane sulfonique ou orotique.

10. Procédé pour la fabrication de composés de la formule générale 2 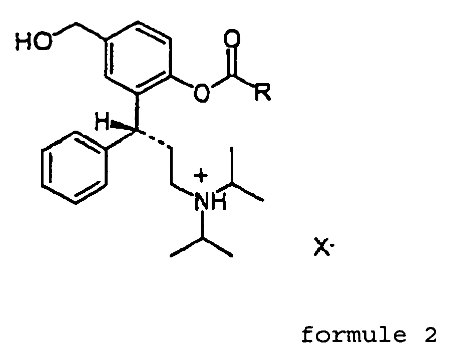 dans laquelle R représente un alkyle en C₁ à C₆, un cycloalkyle en C₃ à C₁₀, un phényle substitué ou non substitué et X⁻ est le résidu acide d'un acide inorganique ou organique physiologiquement compatible, **caractérisé en ce que**
a) un composé de la formule 3 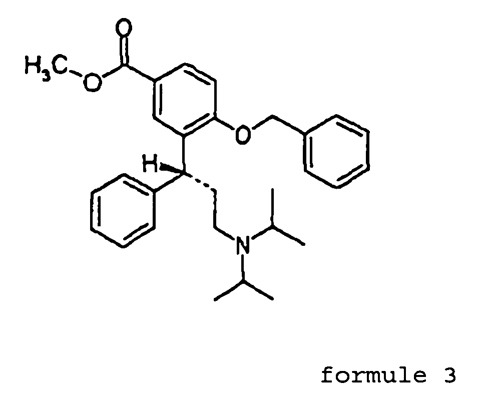 est décomposé avec un hydrogénant pour former un composé de la formule 5 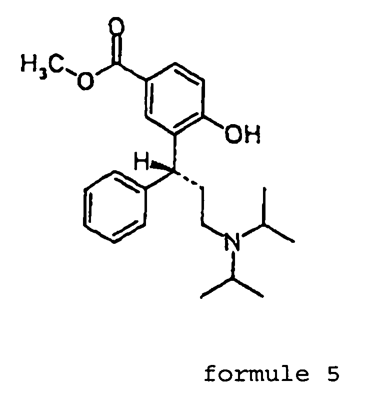 après quoi
b) le composé ainsi obtenu de la formule 5 est transformé avec un agent réducteur pour donner un composé de la formule 6 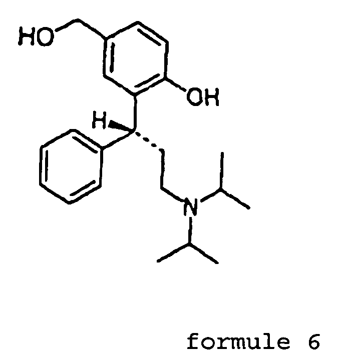 qui
c) est transformé avec un agent d'acylation pour donner un composé de la formule 1 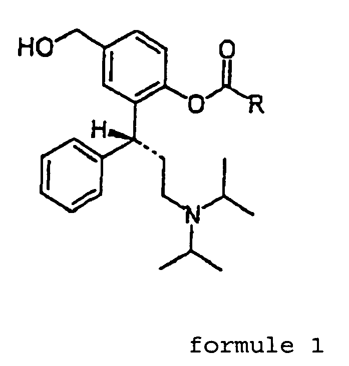 dans laquelle R a la signification définie ci-dessus, composé qui
d) est transformé avec un acide inorganique ou organique physiologiquement compatible moyennant la formation d'un composé de la formule 2
e) 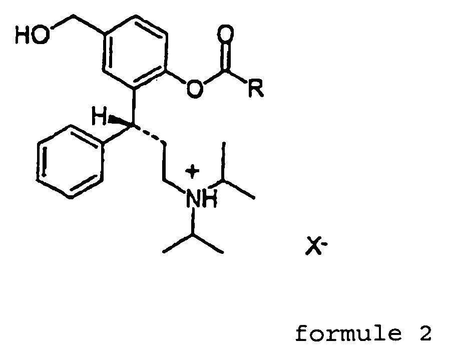
dans laquelle R représente un alkyle en C₁ à C₆, un cycloalkyle en C₃ à C₁₀, un phényle substitué ou non substitué et X⁻ le résidu acide d'un acide inorganique ou organique physiologiquement compatible.

11. Procédé selon la revendication 10, **caractérisé en ce que**, pour la fabrication des composés de la formule générale 2, sont utilisés les acides : chlorhydrique, bromhydrique, phosphorique, sulfurique, nitrique, acétique, propionique, palmitique, stéarique, maléique, fumarique, oxalique, succinique, malique DL, malique L (-), malique D (+), tartrique DL, tartrique L (+), tartrique D (-), citrique, aspartique L, ascorbique L (+), glucuronique D (+), 2-oxopropionique (acide pyruvique), furanne-2-carboxylique (acide mucique), benzoïque, 4-hydroxybenzoïque, salicylique, vanillique, 4-hydroxycinnamique, gallique, hippurique (N-benzoyl-glycine), acéturique (N-acétylglycine), phlorétique (acide 3-(4-hydroxyphényl)propionique), phtalique, méthane sulfonique ou orotique.

12. Procédé selon les revendications 8 à 11, **caractérisé en ce que** le nickel de Raney et H₂ sont utilisés comme agents d'hydrogénation, dans du méthanol comme solvant.

13. Procédé selon les revendications 8 à 11, **caractérisé en ce que** comme agents réducteurs sont utilisés NaBH_{4/}EtOH, de préférence LiAlH₄/THF.

14. Procédé selon les revendications 8 à 11, **caractérisé en ce que** comme agent d'acylation est utilisé du chlorure d'isobutyryle et comme base la triéthylamine.

15. Procédé selon les revendications 10 à 14, **caractérisé en ce qu'**un composé de la formule 6, avec un équivalent de chlorure d'isobutyryle en présence de triéthylamine moyennant l'utilisation d'un des solvants respectifs : acétate d'éthyle, dichlorométhane, tétrahydrofuranne, acétonitrile ou toluène, est transformée de façon régio- et chimiosélective en ester isobutyrique de R-(+)-2-(3-diisopropylamino-1-phénylpropyl)-4-hydroxyméthylphényle.

16. Procédé selon les revendications 10 à 15, **caractérisé en ce que** l'ester isobutyrique de R-(+)-2-(3-diisopropylamino-1-phénylpropyl)-4-hydroxyméthylphényle et l'acide fumarique ou l'acide chlorhydrique réagissent moyennant la formation du sel correspondant.

17. Procédé selon les revendications 10 à 13 pour fabriquer du chlorhydrate hydraté de l'ester isobutyrique de R-(+)-2-(3-diisopropylamino-1-phénylpropyl)-4-hydroxyméthylphényle, **caractérisé en ce que** l'estérification phénolique du R-(+)-2-(3-diisopropylamino-1-phénylpropyl)-4-hydroxyméthylphénol (6) est effectuée sans l'addition d'une base externe, **en ce que** des solutions de (6) sont introduites goutte à goutte dans des solutions de chlorure d'isobutyryle qui contiennent au moins un équivalent molaire d'eau, pour obtenir directement un chlorhydrate hydraté stable correspondant.
